# EUROPÄISCHE PATENTSCHRIFT

(11) **EP 1 533 303 B1**
(45) Veröffentlichungstag und Bekanntmachung des Hinweises auf die Patenterteilung: **24.04.2013**
(21) Anmeldenummer: 05002366.2
(22) Anmeldetag: 02.03.1998
(51) Int. Cl.: A01N 37/46, A01N 43/40, A01N 43/54, A01N 43/82, C07C 237/44, C07D 239/34, C07D 239/54, C07D 239/52, C07D 239/553, C07D 285/08, C07D 413/12

(54) **Substituierte Aminosalicylsäureamide ( Zwischenprodukte)**
Substituted Aminosalicylacid Amides ( intermediates )
Dérivés substitués de l'amiiosalicylacid amides ( produits intermédiaires )

(30) Priorität: 14.03.1997 DE 19710609
(43) Veröffentlichungstag der Anmeldung: 25.05.2005
(62) Teilanmeldung aus: 98913618.9
(73) Patentinhaber: Bayer CropScience AG, 40789 Monheim (DE)
(72) Erfinder: Seitz, Thomas, 40764 Langenfeld (DE); Stelzer, Uwe, 51399 Burscheid (DE); Wolfrum, Peter, 40789 Monheim (DE); Stenzel, Klaus, 40595 Düsseldorf (DE)

(56) Entgegenhaltungen:
- J.Chem.Soc., 1955, Seiten 3478-33481, XP009045688 London
- PATENT ABSTRACTS OF JAPAN Bd. 011, Nr. 038 (C-401), 4. Februar 1987 (1987-02-04) & JP 61 205262 A (DAIKIN IND LTD), 11. September 1986 (1986-09-11)
- J.Fluorine Chem., Bd. 45, 1989, Seiten 417-430, XP002322252 London
- Chem.Pharm.Bull., Bd. 39, Nr. 9, 1991, Seiten 2308-2315, XP002322253 Tokyo
- Pharm.Chem.J.., Bd. 23, Nr. 6, 1989, Seiten 500-503, XP009045680 London

## Beschreibung

Die Erfindung betrifft neue substituierte Aminosalicylsäureamide, mehrere Verfahren zu ihrer Herstellung und ihre Verwendung als Fungizide, sowie neue Zwischenprodukte und mehrere Verfahren zu deren Herstellung.

Bestimmte substituierte Acylaminosalicylsäureamide, wie beispielsweise die Verbindungen 3-Formamido-salicylanilid und 3-(Formylamino)-2-hydroxy-N-(phenyl-methyl)-benzamid, sind bereits bekannt (vergleiche z. B. Biochim. Biophys. Acta (1993), 1142(3), 262-8, J. Med. Chem. (1990), 33(1), 136-42 oder J. Biol. Chem. (1971), 246(23), 7125-30). Eine Wirkung gegen Schädlinge ist von diesen vorbekannten Verbindungen bisher jedoch nicht beschrieben.

Neu, und Gegenstand der vorliegenden Anmeldung, sind Amine der Formel 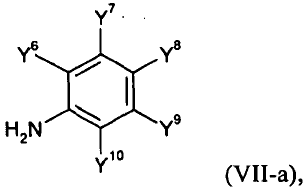 in welcher
- Y⁶, Y⁹ und Y¹⁰: gleich oder verschieden sind und für Wasserstoff, Halogen, Cyano, Alkyl, Halogenalkyl, Alkoxy oder Halogenalkoxy mit jeweils 1-4 Kohlenstoffatomen stehen,
- Y⁷ und Y⁸: verschieden sind und und für Wasserstoff, Halogen, Cyano, Alkyl, Halogenalkyl, Alkoxy oder Halogenalkoxy mit jeweils 1-4 Kohlenstoffatomen stehen, und einer der Reste
- Y⁷ oder Y⁸: für eine Gruppierung 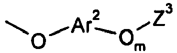 steht,
worin
m für 0 oder 1 steht,
Ar² für 1,2,4-Oxadiazol-3,5-diyl, 1,2,4-Thiadiazol-3,5-diyl oder für gegebenenfalls in 5-Stellung durch Halogen substituiertes Pyrimidin-4,6-diyl steht und
Z³ für gegebenenfalls substituiertes Aryl steht.

Bevorzugt sind Amine der Formel (VII-a),
in welcher
- Y⁶, Y⁹ und Y¹⁰: gleich oder verschieden sind und für Wasserstoff, Halogen, Cyano, Alkyl, Halogenalkyl, Alkoxy oder Halogenalkoxy mit jeweils 1 bis 4 Kohlenstoffatomen stehen,
- Y⁷ und Y⁸: verschieden sind und und für Wasserstoff, Halogen, Cyano, Alkyl, Halogenalkyl, Alkoxy oder Halogenalkoxy mit jeweils 1-4 Kohlenstoffatomen stehen,
und einer der Reste
- Y⁷ oder Y⁸: für eine Gruppierung 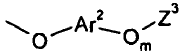 steht,
worin
m für 0 oder 1 steht,
Ar² für 1,2,4-Oxadiazol-3,5-diyl, 1,2,4-Thiadiazol-3,5-diyl oder für gegebenenfalls in 5-Stellung durch Fluor oder Chlor substituiertes Pyrimidin-4,6-diyl steht und
Z³ für jeweils gegebenenfalls einfach oder mehrfach, gleich oder verschieden substituiertes Phenyl oder Naphthyl steht, wobei die möglichen Substituenten vorzugsweise aus der nachstehenden Aufzählung ausgewählt sind:
Halogen, Cyano, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 6 Kohlenstoff atomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 11 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl oder Alkylsulfonyloxy, mit jeweils 1 bis 6 Kohlenstoffatomen in den einzelnen Alkylteilen;
jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen;
Cycloalkyl mit 3 bis 6 Kohlenstoffatomen;
jeweils gegebenenfalls einfach oder mehrfach, gleich oder verschieden durch Halogen und/oder geradkettiges oder verzweigtes Alkyl mit 1 bis 4 Kohlenstoffatomen substituiertes Phenyl oder Phenoxy;
Heterocyclyl oder Heterocyclyl-methyl mit jeweils 3 bis 7 Ringgliedern, von denen jeweils 1 bis 3 gleiche oder verschiedene Heteroatome sind - insbesondere Stickstoff, Sauerstoff und/oder Schwefel - , oder
eine Gruppierung 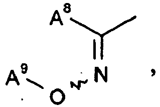 worin
   A⁸ für Alkyl mit 1 bis 4 Kohlenstoffatomen oder Cycloalkyl mit 1 bis 6 Kohlenstoffatomen steht und
   A⁹ für gegebenenfalls durch Cyano, Alkoxy, Alkylthio, Alkylamino, Dialkylamino oder Phenyl substituiertes Alkyl mit 1 bis 4 Kohlenstoffatomen, Alkenyl oder Alkinyl mit jeweils 2 bis 4 Kohlenstoffatomen steht, oder
eine Gruppierung 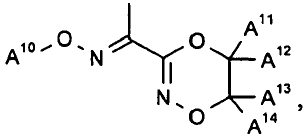 in welcher
   A¹⁰ für Alkyl mit 1 bis 4 Kohlenstoffatomen steht und
   A¹¹, A¹², A¹³ und A¹⁴ gleich oder verschieden sind und unabhängig voneinander für Wasserstoff, Alkyl oder Hydroxyalkyl mit jeweils 1 bis 4 Kohlenstoffatomen oder Halogenalkyl mit 1 bis 4 Kohlenstoffatomen und 1 bis fünf gleichen oder verschiedenen Halogenatomen stehen, oder
   A¹¹ und A¹² oder A¹¹ und A¹³ oder A¹³ und A¹⁴ gemeinsam mit den jeweiligen Kohlenstoffatomen, an die sie gebunden sind, einen cycloaliphatischen Ring mit fünf, sechs oder sieben Kohlenstoffatomen bilden.

Besonders bevorzugt sind Amine der Formel (VII-a),
in welcher
- Y⁶, Y⁹ und Y¹⁰: gleich oder verschieden sind und für Wasserstoff, Fluor, Chlor, Brom, Cyano, Methyl, Ethyl, Methoxy, Trifluormethyl oder Difluormethoxy stehen, und
- Y⁷ und Y⁸: verschieden sind und für Wasserstoff, Fluor, Chlor, Brom, Cyano, Methyl, Ethyl, Methoxy, Trifluormethyl oder Difluormethoxy stehen, und einer der Reste
- Y⁷ oder Y⁸: für eine Gruppierung 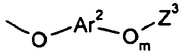 steht,
worin
m für 0 oder 1 steht,
Ar² für 1,2,4-Oxadiazol-3,5-diyl, 1,2,4-Thiadiazol-3,5-diyl oder für gegebenenfalls in 5-Stellung durch Fluor oder Chlor substituiertes Pyrimidin-4,6-diyl steht und
Z³ für jeweils gegebenenfalls einfach bis dreifach, gleich oder verschieden substituiertes Phenyl steht, wobei die möglichen Substituenten vorzugsweise aus der nachstehenden Aufzählung ausgewählt sind:
Fluor, Chlor, Brom, Cyano, Methyl, Ethyl, n- oder i-Propyl, n-, i-, s-oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Trifluormethylthio, Difluorchlormethylthio, Trifluormethylsulfinyl oder Trifluormethylsulfonyl, Methoxycarbonyl, Ethoxycarbonyl,
jeweils gegebenenfalls einfach bis vierfach, gleich oder verschieden durch Fluor, Chlor, Methyl, Trifluormethyl oder Ethyl substituiertes, jeweils zweifach verknüpftes Methylendioxy, Ethylendioxy
Phenyl, 4-Chlorphenyl, 4-Methylphenyl, Phenoxy, 4-Chlorphenoxy, 4-Methylphenoxy, Cyclopropyl, Cyclobutyl, Cyclopentyl oder Cyclohexyl, oder
eine Gruppierung 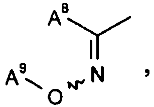 worin
   A⁸ für Methyl, Ethyl, n- oder i-Propyl, n-, i-, s- oder t-Butyl, Cyclopropyl oder Cyclobutyl steht,
   A⁹ für Methyl, Ethyl, n- oder i-Propyl, n-, i-, s- oder t-Butyl, Allyl, Propargyl, But-2-en-1-yl, 2-Methyl-prop-1-en-3-yl, Cyanmethyl, Methoxymethyl, Ethoxymethyl, Methoxyethyl, Ethoxyethyl, Methylthiomethyl, Ethylthiomethyl, Methylthioethyl, Ethylthioethyl, Dimethylaminomethyl, Dimethylaminoethyl, Methylaminomethyl, Methylaminoethyl oder Benzyl steht, oder
eine Gruppierung 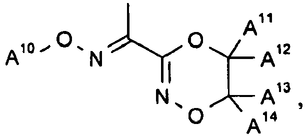 in welcher
   A¹⁰ für Methyl oder Ethyl steht und
   A¹¹, A¹², A¹³ und A¹⁴ gleich oder verschieden sind und unabhängig voneinander für Wasserstoff, Methyl, Ethyl, n- oder i-Propyl, n-, i-, s- oder t-Butyl, Hydroxymethyl, Trifluormethyl oder Trifluorethyl stehen, oder
   A¹¹ und A¹² oder A¹¹ und A¹³ oder A¹³ und A¹⁴ gemeinsam mit den jeweiligen Kohlenstoffatomen, an die sie gebunden sind, einen cycloaliphatischen Ring mit fünf, sechs oder sieben Kohlenstoffatomen bilden.

Die Amine der Formel (VII-a) werden erhalten (Verfahren h), wenn man Diazole der allgemeinen Formel (VIII) oder Halogenpyrimidine der allgemeinen Formel (IX), 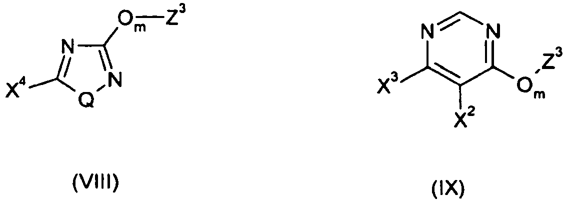 worin
- m: für 0 oder 1 steht,
- X²: für Wasserstoff, Fluor oder Chlor steht,
- X³: für Chlor oder Fluor steht,
- X⁴: für Methylsulfonyl, Chlor oder Brom steht und
- Q: für Sauerstoff oder Schwefel steht, und
- Z³: die oben angegebene Bedeutung hat,
mit einem Aminophenol der Formel, 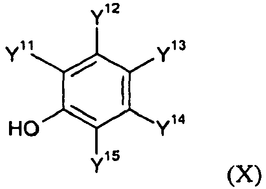 in welcher
- Y¹¹, Y¹⁴ und Y¹⁵: gleich oder verschieden sind und und für Wasserstoff, Halogen, Cyano, Alkyl, Halogenalkyl, Alkoxy oder Halogenalkoxy mit jeweils 1-4 Kohlenstoffatomen stehen,
- Y¹² oder Y¹³: verschieden sind und für Wasserstoff, Halogen, Cyano, Alkyl, Halogenalkyl, Alkoxy oder Halogenalkoxy mit jeweils 1-4 Kohlenstoffatomen stehen, und einer der Reste
- Y¹² oder Y¹³: für Amino steht,
gegebenenfalls in Gegenwart eines Verdünnungsmittels, gegebenenfalls in Gegenwart eines Säureakzeptors und gegebenenfalls in Gegenwart eines Katalysators, umsetzt, oder wenn man (Verfahren i) Aminophenoxypyrimidine der allgemeinen Formel (XI), 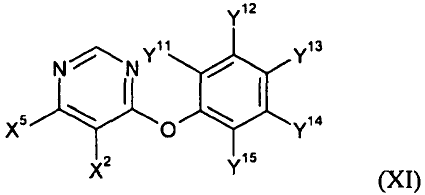 in welcher
- X², Y¹¹, Y¹², Y¹³, Y¹⁴ und Y¹⁵: die oben angegebenen Bedeutungen haben und
- X⁵: für Chlor oder Fluor steht,
mit einem Phenol der allgemeinen Formel (XII),

Z³-OH (XII)

in welcher
- Z³: die oben angegebene Bedeutung hat,
gegebenenfalls in Gegenwart eines Verdünnungsmittels, gegebenenfalls in Gegenwart eines Säureakzeptors und gegebenenfalls in Gegenwart eines Katalysators, umsetzt.

Die zur Durchführung des erfindungsgemäßen Verfahrens h) als Ausgangsstoffe benötigten Diazole sind durch die Formel (VIII), allgemein definiert. In dieser Formel (VIII) haben m und Z³ vorzugsweise bzw. insbesondere diejenigen Bedeutungen, die bereits im Zusammenhang mit der Beschreibung der erfindungsgemäßen Verbindungen der Formel (VII-a) als bevorzugt bzw. als insbesondere bevorzugt für m und Z³ angegeben wurden. X⁴ steht für Methylsulfonyl, Chlor oder Brom.

Die Diazole der Formel (VIII) sind bekannt und können nach bekannten Methoden hergestellt werden (vergleiche z. B. DE-A 2142913).

Die zur Durchführung des erfindungsgemäßen Verfahrens h) als Ausgangsstoffe alternativ benötigten Halogenpyrimidine sind durch die Formel (IX), allgemein definiert. In dieser Formel (IX) haben m und Z³ vorzugsweise bzw. insbesondere diejenigen Bedeutungen, die bereits im Zusammenhang mit der Beschreibung der erfindungsgemäßen Verbindungen der Formel (VII-a) als bevorzugt bzw. als insbesondere bevorzugt für m und Z³ angegeben wurden. X² steht für Wasserstoff, Fluor oder Chlor. X³ steht für Fluor oder Chlor.

Die Halogenpyrimidine der Formel (IX) sind teilweise bekannt und können nach bekannten Methoden hergestellt werden (vgl. z.B. Pharm.Chem.J.(Engl.Transl.), 23, 6, 1989, 500-503; RU, 23, 6, 1989, 705-707).

Neu, und auch Gegenstand der vorliegenden Anmeldung, sind 4-Chlor-5-fluor-6-phenoxypyrimidine der allgemeinen Formel 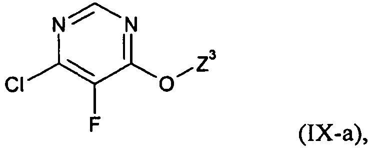 in welcher
- Z³: oben angegebene Bedeutung hat, wobei die Verbindung 2-[(6-Chlor-5-fluor-4-pyrimidinyl)-oxy]-α-(methoxymethylen)-benzoesäuremethylester, ausgenommen ist.

Die neuen 4-Chlor-5-fluor-6-phenoxypyrimidine werden erhalten, wenn man (Verfahren j) Phenole der allgemeinen Formel (XII),

Z³-OH (XII)

in welcher
- Z³: die oben angegebene Bedeutung hat,
mit 4,6-Dichlor-5-fluorpyrimidin (XIII), gegebenenfalls in Gegenwart eines Verdünnungsmittels, gegebenenfalls in Gegenwart eines Säureakzeptors und gegebenenfalls in Gegenwart eines Katalysators, umsetzt.

Die zur Durchführung des erfindungsgemäßen Verfahrens j) als Ausgangsstoffe benötigten Phenole sind durch die Formel (XII) allgemein definiert. In dieser Formel (XII) hat Z³ vorzugsweise bzw. insbesondere diejenige Bedeutung, die bereits im Zusammenhang mit der Beschreibung der erfindungsgemäßen Verbindungen der Formel (VII-a) als bevorzugt bzw, als insbesondere bevorzugt für Z³ angegeben wurde.

Die Phenole der Formel (XII) sind bekannte Synthesechemikalien oder können nach bekannten Verfahren hergestellt werden (WO 95-04728).

Das zur Durchführung des erfindungsgemäßen Verfahrens j) als Ausgangsstoff weiterhin benötigte 4,6-Dichlor-5-fluorpyrimidin (XIII) 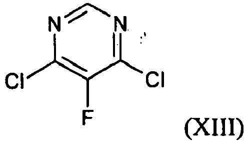 ist neu und ebenfalls Gegenstand der vorliegenden Anmeldung.

Es wird erhalten, wenn man (Verfahren k) 5-Fluor-6-hydroxy-4(1H)-pyrimidinon (XIV) mit einem Chlorierungsmittel, gegebenenfalls in Gegenwart eines Verdünnungsmittels und gegebenenfalls in Gegenwart eines Katalysators, umsetzt.

Das zur Durchführung des erfindungsgemäßen Verfahrens k) als Ausgangsstoff benötigte 5-Fluor-6-hydroxy-4(1H)-pyrimidinon (XIV) ist bekannt und kann nach bekannten Methoden hergestellt werden (JP 61205262; CA: 106:84632).

Die zur Durchführung des erfindungsgemäßen Verfahrens i) als Ausgangsstoffe benötigten Aminophenoxypyrimidine sind durch die Formel (XI) allgemein definiert. In dieser Formel (XI) haben Y¹¹, Y¹², Y¹³, Y¹⁴ und Y¹⁵ vorzugsweise bzw. insbesondere diejenigen Bedeutungen, die bereits im Zusammenhang mit der Beschreibung der Amine der Formel (X) als bevorzugt bzw. als insbesondere bevorzugt für Y¹¹, Y¹², Y¹³, Y¹⁴ und Y¹⁵ angegeben wurden. X² und X⁵ stehen unabhängig voneinander für Chlor oder Fluor.

Die Aminophenoxypyrimidine der Formel (XI) 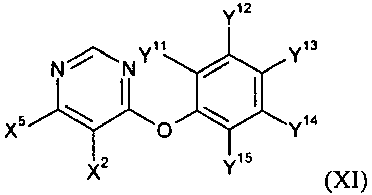 in welcher
- X², Y¹¹, Y¹², Y¹³, Y¹⁴ und Y¹⁵: die oben angegebenen Bedeutungen haben und
- X⁵: für Chlor oder Fluor steht,
sind neu und ebenfalls Gegenstand der vorliegenden Anmeldung.

Sie werden erhalten, wenn man (Verfahren 1) Trihalogenpyrimidine der Formel (XV), 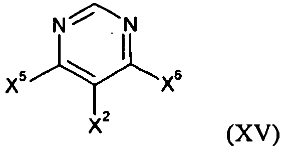 in welcher
- X² und X⁵: die oben angegebenen Bedeutungen haben und
- X⁶: für Fluor oder Chlor steht,
mit einem Aminophenol der Formel (X),
gegebenenfalls in Gegenwart eines Verdünnungsmittels, gegebenenfalls in Gegenwart eines Säureakzeptors und gegebenenfalls in Gegenwart eines Katalysators, umsetzt.

Die zur Durchführung des erfindungsgemäßen Verfahrens 1) als Ausgangsstoffe benötigten Trihalogenpyrimidine sind durch die Formel (XV) allgemein definiert. In dieser Formel (XV) haben X² und X⁵ vorzugsweise bzw. insbesondere diejenigen Bedeutungen, die bereits im Zusammenhang mit der Beschreibung der Verbindungen der Formel (XI) als bevorzugt bzw. als insbesondere bevorzugt für X² und X⁵ angegeben wurden. X⁶ steht für Fluor oder Chlor.

Die Trihalogenpyrimidine der Formel (XV) sind teilweise bekannt und können nach bekannten Methoden hergestellt werden (vergleiche z. B. Chesterfield et al., J. Chem. Soc., 1955; 3478, 3480). Ein Sonderfall der Verbindungen der Formel (XV) ist die erfindungsgemäße Verbindung (XIII); sie kann nach Verfahren k) hergestellt werden.

Die zur Durchführung des erfindungsgemäßen Verfahrens 1) weiterhin als Ausgangsstoffe benötigten Aminophenole der Formel (X) sind bereits weiter oben bei der Beschreibung des erfindungsgemäßen Verfahrens h) beschrieben worden.

Die zur Durchführung des erfindungsgemäßen Verfahrens i) weiterhin als Ausgangsstoffe benötigten Phenole der Formel (XII) sind bereits weiter oben bei der Beschreibung des erfindungsgemäßen Verfahrens j) beschrieben worden.

Die zur Durchführung des erfindungsgemäßen Verfahrens e) als Ausgangsstoffe benötigten O-Benzyl-nitrosalicylsäureamide sind durch die Formel (V), allgemein definiert. In dieser Formel (V) haben Y¹, Y², Y³, Y⁴ und Y⁵ vorzugsweise bzw. insbesondere diejenigen Bedeutungen, die bereits im Zusammenhang mit der Beschreibung der erfindungsgemäßen Verbindungen der Formel (I) als bevorzugt bzw. als insbesondere bevorzugt für Y¹, Y², Y³, Y⁴ und Y⁵ angegeben wurden.

Die O-Benzyl-nitrosalicylsäureamide der Formel (V) sind noch nicht bekannt, sie sind als neue Stoffe Gegenstand der vorliegenden Anmeldung.

Die O-Benzyl-nitrosalicylsäureamide der Formel (V) werden erhalten, wenn man (Verfahren g) O-Benzyl-nitrosalicylsäurederivate der Formel (VI), 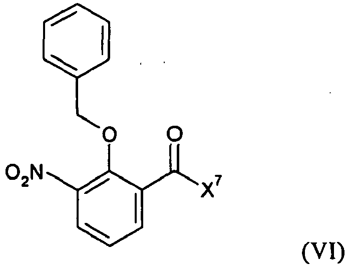 in welcher
- X⁷: für Halogen, Hydroxy oder Alkoxy steht,
mit einem Amin der Formel (VII),
gegebenenfalls in Gegenwart eines Verdünnungsmittels, gegebenenfalls in Gegenwart eines Kondensationsmittel und gegebenenfalls in Gegenwart eines Säureakzeptors, umsetzt.

Die zur Durchführung des erfindungsgemäßen Verfahrens g) als Ausgangsstoffe benötigten O-Benzyl-nitrosalicylsäurederivate sind durch die Formel (VI), allgemein definiert. In dieser Formel (VI) steht X⁷ für Halogen, vorzugsweise Chlor; Hydroxy oder Alkoxy, vorzugsweise Methoxy oder Ethoxy.

Die O-Benzyl-nitrosalicylsäurederivate der Formel (VI) sind bekannt und können nach bekannten Methoden hergestellt werden (vergleiche z. B. J. Am. Chem. Soc. 1959, 5215-5217).

Die zur Durchführung des erfindungsgemäßen Verfahrens g) weiterhin als Ausgangsstoffe benötigten Amine der Formel (VII) bereits weiter oben im Zusammenhang mit der Beschreibung des erfindungsgemäßen Verfahrens f) beschrieben worden.

Die zur Durchführung des erfindungsgemäßen Verfahrens a) weiterhin als Ausgangsstoffe benötigten Acylierungsmittel sind durch die Formel (III), allgemein definiert. In dieser Formel (III) hat R¹ vorzugsweise bzw. insbesondere diejenige Bedeutung, die bereits im Zusammenhang mit der Beschreibung der erfindungsgemäßen Verbindungen der Formel (I) als bevorzugt bzw. als insbesondere bevorzugt für R¹ angegeben wurde. X¹ steht für Halogen, Hydroxy, Alkoxy oder Alkylcarbonyloxy, vorzugsweise für Chlor, Hydroxy, Methoxy, Ethoxy oder Acetoxy.

Die Acylierungsmittel der allgemeinen Formel (III) sind bekannte Reagenzien in der organischen Chemie.

Die zur Durchführung des erfindungsgemäßen Verfahrens b) als Ausgangsstoffe benötigten Nitrosalicylsäureamide der Formel (IV) sind bereits weiter oben im Zusammenhang mit der Beschreibung des erfindungsgemäßen Verfahrens d) beschrieben worden.

Die zur Durchführung des erfindungsgemäßen Verfahrens c) als Ausgangsstoffe benötigten O-Benzyl-nitrosalicylsäureamide der Formel (V) sind bereits weiter oben im Zusammenhang mit der Beschreibung des erfindungsgemäßen Verfahrens e) beschrieben worden.

Als Verdünnungsmittel zur Durchführung der erfindungsgemäßen Verfahren a), f) und g) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid oder Sulfone, wie Sulfolan.

Als Verdünnungsmittel zur Durchführung der erfindungsgemäßen Verfahren d)und e) kommen alle inerten organischen Lösungsmittel in Betracht. Hierzu gehören vorzugsweise Ester wie Essigsäuremethylester oder Essigsäureethylester; Alkohole, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether, Wasser, Salzlösungen, wie beispielsweise Ammoniumchloridlösung, Säuren, wie beispielsweise Salzsäure oder Essigsäure, sowie beliebige Mischungen der genannten Verdünnungsmittel.

Als Verdünnungsmittel zur Durchführung der erfindungsgemäßen Verfahren h), i), j) und 1) kommen alle inerten organischen Lösungsmittel in Betracht. Hierzu gehören vorzugsweise Ether, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Sulfoxide, wie Dimethylsulfoxid; oder Sulfone, wie Sulfolan.

Als Verdünnungsmittel zur Durchführung des erfindungsgemäßen Verfahrens k) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan;| Nitrile, wie Acetonitril, Propionitril, n- oder i-Butyronitril oder Benzonitril.

Die erfindungsgemäßen Verfahren a), f) und g) werden gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydroxide, -acetate, -carbonate oder -hydrogencarbonate, wie beispielsweise Natriumhydroxid, Kaliumhydroxid, Natriumacetat, Kaliumacetat, Calciumacetat, Natriumcarbonat, Kaliumcarbonat, Kaliumhydrogencarbonat oder Natriumhydrogencarbonat sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die erfindungsgemäßen Verfahren b), c), d) und e) werden gegebenenfalls in Gegenwart eines Katalysators durchgerührt. Als solche kommen alle Katalysatoren infrage, die auch für Hydrierungen üblicherweise verwendet werden. Beispielhaft seien genannt: Raney-Nickel, Palladium oder Platin, gegebenenfalls auf einem Trägermaterial, wie beispielsweise Aktivkohle.

Das erfindungsgemäße Verfahren c) wird gegebenenfalls auch in Gegenwart von Wasserstoff oder gegebenenfalls in Gegenwart eines unedlen Metalls durchgeführt.

Als unedle Metalle seien beispielhaft genannt: Zink, Zinn, Eisen, Aluminium oder Magnesium.

Als weitere Reaktionshilfsmittel zur Durchführung der erfindungsgemäßen Verfahren a), b) und c) kommen alle wasserentziehenden Mittel, insbesondere Essigsäureanhydrid, infrage.

Die erfindungsgemäßen Verfahren f) und g) werden gegebenenfalls in Gegenwart eines Kondensationsmittels durchgeführt. Hierzu gehören vorzugsweise Säurehalogenidbildner wie beispielsweise Phosgen, Phosphortribromid, Phosphortrichlorid, Phosphorpentachlorid, Phosphoroxychlorid oder Thionylchlorid; Anhydridbildner wie Chlorameisensäureethylester, Chlorameisensäuremethylester, Chlorameisensäureisopropylester, Chlorameisensäureisobutylester oder Methansulfonylchlorid; Carbodiimide, wie N,N'-Dicyclohexylcarbodiimid (DCC) oder andere übliche Kondensationsmittel, wie Phosphorpentoxid, Polyphosphorsäure, N,N'-Carbonyldiimidazol, 2-Ethoxy-N-ethoxycarbonyl-1,2-dihydrochinolin (EEDQ) oder Triphenylphosphin/Tetrachlormethan.

Die erfindungsgemäßen Verfahren h), i), j) und 1) werden gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydride, -hydroxide, -alkoholate, -carbonate oder -hydrogencarbonate, wie beispielsweise Natriumhydrid, Natriumamid, Kalium-tert.-butylat, Natriumhydroxid, Kaliumhydroxid, Natriumcarbonat, Kaliumcarbonat, Kaliumhydrogencarbonat oder Natriumhydrogencarbonat.

Als Katalysatoren für die erfindungsgemäßen Verfahren h), i), j) und 1) eignen sich alle Kupfer(I)-Salze, wie beispielsweise Kupfer(I)-chlorid, Kupfer(I)-bromid oder Kupfer(I)-iodid, oder auch Fluoride, wie beilspielsweise Natrium-, Kalium- oder Ammoniumfluorid, sowie tertiäre Ammoniumfluoride, wie Tetrabutylammoniumfluorid.

Das erfindungsgemäße Verfahren k) wird gegebenenfalls in Gegenwart eines geeigneten Katalysators durchgeführt. Als solche kommen alle tertiäre Amine infrage, wie beispielsweise Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU), sowie Amide, wie Dimethylformamid.

Als Halogenierungsmittel zur Durchführung des erfindungsgemäßen Verfahrens k) kommen alle Reagenzien infrage, die an Kohlenstoff gebundene Hydroxygruppen gegen Chlor austauschen können. Beispielhaft seien genannt: Phosgen, Phosphortrichlorid, Phosphorpentachlorid, Phosphoroxychlorid oder Thionylchlorid und gegebenenfalls zusätzlich Chlorwasserstoff oder Chlor.

Die Reaktionstemperaturen können bei der Durchführung der erfindungsgemäßen Verfahren a), b), c), d), e), f) und g) in einem größeren Bereich variiert werden. Im allgemeinen arbeitet man bei Temperaturen von 0°C bis 180°C, vorzugsweise bei Temperaturen von 0°C bis 130°C.

Die Reaktionstemperaturen können bei der Durchführung der erfindungsgemäßen Verfahren h), i), j) und l) in einem größeren Bereich variiert werden. Im allgemeinen arbeitet man bei Temperaturen von -20°C bis 200°C, vorzugsweise bei Temperaturen von -10°C bis 150°C.

Die Reaktionstemperaturen können bei der Durchführung des erfindungsgemäßen Verfahrens k) in einem größeren Bereich variiert werden. Im allgemeinen arbeitet man bei Temperaturen von 0°C bis 250°C, vorzugsweise bei Temperaturen von 0°C bis 200°C.

Zur Durchführung des erfindungsgemäßen Verfahrens a) zur Herstellung der Verbindungen der Formel (I) setzt man pro Mol des Aminosalicylsäureamids der Formel (II) im allgemeinen 1 bis 2000 Mol, vorzugsweise 1 bis 800 Mol Acylierungsmittel der Formel (III) ein.

Zur Durchführung der erfindungsgemäßen Verfahren b) und c) zur Herstellung der Verbindungen der Formel (I) setzt man pro Mol des Nitrosalicylsäureamides der Formel (IV), bzw. des O-Benzyl-nitrosalicylsäureamides der Formel (V) im allgemeinen 100 bis 2000 Mol, vorzugsweise 200 bis 1000 Mol Ameisensäure ein.

Zur Durchführung des erfindungsgemäßen Verfahrens d) zur Herstellung der Verbindungen der Formel (II) setzt man pro Mol des Nitrosalicylsäureamides der Formel (IV) im allgemeinen 1 bis 100 Mol, vorzugsweise 2 bis 20 Mol Reduktionsmittel ein.

Zur Durchführung des erfindungsgemäßen Verfahrens e) zur Herstellung der Verbindungen der Formel (II) setzt man pro Mol des O-Benzyl-nitrosalicylsäureamides der Formel (V) im allgemeinen 1 bis 100 Mol, vorzugsweise 2 bis 50 Mol Wasserstoff ein.

Zur Durchführung des erfindungsgemäßen Verfahrens f) zur Herstellung der Verbindungen der Formel (IV) setzt man pro Mol Amin der Formel (VII) im allgemeinen 0,5 bis 10 Mol, vorzugsweise 0,8 bis 5 Mol 2-Hydroxy-3-nitrobenzoesäure oder 2-Hydroxy-3-nitrobenzoylchlorid ein.

Zur Durchführung des erfindungsgemäßen Verfahrens g) zur Herstellung der Verbindungen der Formel (V) setzt man pro Mol des Amins der Formel (VII) im allgemeinen 0,5 bis 10 Mol, vorzugsweise 0,8 bis 5 Mol O-Benzyl-nitrosalicylsäurederivate der Formel (VI) ein.

Zur Durchführung des erfindungsgemäßen Verfahrens h) zur Herstellung der Verbindungen der Formel (VII-a) setzt man pro Mol des Aminophenols der Formel (X) im allgemeinen 0,5 bis 5 Mol, vorzugsweise 0,8 bis 2 Mol Halogenpyrimidin der Formel (IX), bzw. Diazol der Formel (VIII) ein.

Zur Durchführung des erfindungsgemäßen Verfahrens i) zur Herstellung der Verbindungen der Formel (VII-a) setzt man pro Mol des Phenols der Formel (XII) im allgemeinen 0,5 bis 5 Mol, vorzugsweise 0,8 bis 2 Mol Aminophenoxypyrimidin (XI) ein.

Zur Durchführung des erfindungsgemäßen Verfahrens j) zur Herstellung der Verbindungen der Formel (IX-a) setzt man pro Mol des Phenols der Formel (XII) im allgemeinen 0,5 bis 5 Mol, vorzugsweise 0,8 bis 2 Mol 4,6-Dichlor-5-fluorpyrimidin (XIII) ein.

Zur Durchführung des erfindungsgemäßen Verfahrens k) zur Herstellung von 4,6-Dichlor-5-fluorpyrimidin (XIII) setzt man pro Mol 5-Fluor-6-hydroxy-4(1H)-pyrimidinon (XIV) im allgemeinen 0,05 bis 20 Mol, vorzugsweise 0,1 bis 10 Mol Halogenierungsmittel ein.

Zur Durchführung des erfindungsgemäßen Verfahrens 1) zur Herstellung der Verbindungen der Formel (XI) setzt man pro Mol des Aminophenols der Formel (X) im allgemeinen 0,5 bis 5 Mol, vorzugsweise 0,8 bis 2 Mol Trihalogenpyrimidin (XV) ein.

Die erfindungsgemäßen Verfahren a) bis 1) werden im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Die erfindungsgemäßen Stoffe weisen eine starke mikrobizide Wirkung auf und können zur Bekämpfung von unerwünschten Mikroorganismen, wie Fungi und Bakterien, im Pflanzenschutz und im Materialschutz eingesetzt werden.

Fungizide lassen sich Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Krankheiten im Wein-, Obst- und Gemüseanbau, wie beispielsweise gegen Venturia- und Phytophtora-Arten, einsetzen. Mit gutem Erfolg werden auch Getreidekrankheiten, wie beispielsweise, Pseudocercosporella-Arten oder Reiskrankheiten, wie beispielsweise Pyricularia-Arten, bekämpft.

Die erfindungsgemäßen Wirkstoffe eignen sich auch zur Steigerung des Ernteertrages. Sie sind außerdem mindertoxisch und weisen eine gute Pflanzenverträglichkeit auf.

Die Wirkstoffe können in Abhängigkeit von ihren jeweiligen physikalischen und/oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die Wirkstoffe können in Abhängigkeit von ihren jeweiligen physikalischen und/oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Wannnebel-Formulierungen.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen in Frage:
**Fungizide:**
   Aldimorph, Ampropylfos, Ampropylfos-Kalium, Andoprim, Anilazin, Azaconazol, Azoxystrobin,
   Benalaxyl, Benodanil, Benomyl, Benzamacril, Benzamacryl-isobutyl, Bialaphos, Binapacryl, Biphenyl, Bitertanol, Blasticidin-S, Bromuconazol, Bupirimat, Buthiobat,
   Calciumpolysulfid, Capsimycin, Captafol, Captan, Carbendazim, Carboxin, Carvon, Chinomethionat (Quinomethionat), Chlobenthiazon, Chlorfenazol, Chloroneb, Chloropicrin, Chlorothalonil, Chlozolinat, Clozylacon, Cufraneb, Cymoxanil, Cyproconazol, Cyprodinil, Cyprofuram,
   Debacarb, Dichlorophen, Diclobutrazol, Diclofluanid, Diclomezin, Dicloran, Diethofencarb, Difenoconazol, Dimethirimol, Dimethomorph, Diniconazol, Diniconazol-M, Dinocap, Diphenylamin, Dipyrithione, Ditalimfos, Dithianon, Dodemorph, Dodine, Drazoxolon,
   Ediphenphos, Epoxiconazol, Etaconazol, Ethirimol, Etridiazol,
   Famoxadon, Fenapanil, Fenarimol, Fenbuconazol, Fenfuram, Fenitropan, Fenpicionil, Fenpropidin, Fenpropimorph, Fentinacetat, Fentinhydroxyd, Ferbam, Ferimzon, Fluazinam, Flumetover, Fluoromid, Fluquinconazol, Flurprimidol, Flusilazol, Flusulfamid, Flutolanil, Flutriafol, Folpet, Fosetyl-Alminium, Fosetyl-Natrium, Fthalid, Fuberidazol, Furalaxyl, Furametpyr, Furcarbonil, Furconazol, Furconazol-cis, Furmecyclox,
   Guazatin,
   Hexachlorobenzol, Hexaconazol, Hymexazol,
   Imazalil, Imibenconazol, Iminoctadin, Iminoctadinealbesilat, Iminoctadinetriacetat, Iodocarb, Ipconazol, Iprobenfos (IBP), Iprodione, Irumamycin, Isoprothiolan, Isovaledione,
   Kasugamycin, Kresoxim-methyl, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
   Mancopper, Mancozeb, Maneb, Meferimzone, Mepanipyrim, Mepronil, Metalaxyl, Metconazol, Methasulfocarb, Methfuroxam, Metiram, Metomeclam, Metsulfovax, Mildiomycin, Myclobutanil, Myclozolin,
   Nickel-dimethyldithiocarbamat, Nitrothal-isopropyl, Nuarimol,
   Ofurace, Oxadixyl, Oxamocarb, Oxolinicacid, Oxycarboxim, Oxyfenthiin,
   Paclobutrazol, Pefurazoat, Penconazol, Pencycuron, Phosdiphen, Pimaricin, Piperalin, Polyoxin, Polyoxorim, Probenazol, Prochloraz, Procymidon, Propamocarb, Propanosine-Natrium, Propiconazol, Propineb, Pyrazophos, Pyrifenox, Pyrimethanil, Pyroquilon, Pyroxyfur,
   Quinconazol, Quintozen (PCNB),
   Schwefel und Schwefel-Zubereitungen,
   Tebuconazol, Tecloftalam, Tecnazen, Tetcyclacis, Tetraconazol, Thiabendazol, Thicyofen, Thifluzamide, Thiophanate-methyl, Thiram, Tioxymid, Tolclofos-methyl, Tolylfluanid, Triadimefon, Triadimenol, Triazbutil, Triazoxid, Trichlamid, Tricyclazol, Tridemorph, Triflumizol, Triforin, Triticonazol,
   Uniconazol,
   Validamycin A, Vinclozolin, Viniconazol,
   Zarilamid, Zineb, Ziram sowie
   Dagger G,
   OK-8705,
   OK-8801,
   α-(1,1-Dimethylethyl)-β-(2-phenoxyethyl)-1 H-1,2,4-triazol-1-ethanol,
   α-(2,4-Dichlorphenyl)-β-fluor-b-propyl-1H-1,2,4-triazol-1-ethanol,
   α-(2,4-Dichlorphenyl)-β-methoxy-a-methyl-1H-1,2,4-triazol-1-ethanol,
   α-(5-Methyl-1,3-dioxan-5-yl)-β-[[4-(trifluormethyl)-phenyl]-methylen]-1H-1,2,4-triazol-1-ethanol,
   (5RS,6RS)-6-Hydroxy-2,2,7,7-tetramethyl-5-(1H-1,2,4-triazol-l-yl)-3-octanon,
   (E)-a-(Methoxyimino)-N-methyl-2-phenoxy-phenylacetamid,
   {2-Methyl-1-[[[1-(4-methylphenyl)-ethyl]-amino]-carbonyl]-propyl}-carbaminsäure-1-isopropylester
   1-(2,4-Dichlorphenyl)-2-(1H-1,2,4-triazol-1-yl)-ethanon-O-(phenylmethyl)-oxim,
   1-(2-Methyl-1-naphthalenyl)-1H-pyrrol-2,5-dion,
   1-(3,5-Dichlorphenyl)-3-(2-propenyl)-2,5-pyrrolidindion,
   1-[(Diiodmethyl)-sulfonyl]-4-methyl-benzol,
   1-[[2-(2,4-Dichlorphenyl)-1,3-dioxolan-2-yl]-methyl]-1H-imidazol,
   1- [[2-(4-Chlorphenyl)-3-phenyloxiranyl]-methyl]-1H-1,2,4-triazol,
   1-[1-[2-[(2,4-Dichlorphenyl)-methoxy]-phenyl]-ethenyl]-1H-imidazol,
   1-Methyl-5-nonyl-2-(phenylmethyl)-3-pyrrolidinol,
   2',6'-Dibrom-2-methyl-4'-trifluormethoxy-4'-trifluor-methyl-1,3-thiazol-5-carboxanilid,
   2,2-Dichlor-N-[1-(4-chlorphenyl)-ethyl]-1-ethyl-3-methyl-cyclopropancarboxamid,
   2,6-Dichlor-5-(methylthio)-4-pyrimidinyl-thiocyanat,
   2,6-Dichlor-N-(4-trifluormethylbenzyl)-benzamid,
   2,6-Dichlor-N-[[4-(trifluormethyl)-phenyl]-methyl]-benzamid,
   2-(2,3,3-Triiod-2-propenyl)-2H-tetrazol,
   2-[(1-Methylethyl)-sulfonyl]-5-(trichlormethyl)-1,3,4-thiadiazol,
   2-[[6-Deoxy-4-O-(4-O-methyl-β-D-glycopyranosyl)-a-D-glucopyranosyl]-amino]-4-methoxy-1H-pyrrolo[2,3-d]pyrimidin-5-carbonitril,
   2-Aminobutan,
   2-Brom-2-(brommethyl)-pentandinitril,
   2-Chlor-N-(2,3-dihydro-1,1,3-trimethyl-1H-inden-4-yl)-3-pyridincarboxamid,
   2-Chlor-N-(2,6-dimethylphenyl)-N-(isothiocyanatomethyl)-acetamid,
   2-Phenylphenol(OPP),
   3,4-Dichlor-1-[4-(difluormethoxy)-phenyl]-1H-pyrrol-2,5-dion,
   3,5-Dichlor-N-[cyan [(1-methyl-2-propyny!)-oxy]-methyll-benzamid,
   3-(1,1-Dimethylpropyl-1-oxo-1H-inden-2-carbonitril,
   3-[2-(4-Chlorphenyl)-5-ethoxy-3-isoxazolidinyl]-pyridin,
   4-Chlor-2-cyan-N,N-dimethyl-5-(4-methylphenyl)-1H-imidazol-1-sulfonamid,
   4-Methyl-tetrazolo[1,5-a]quinazolin-5(4H)-on,
   8-(1,1-Dimethylethyl)-N-ethyl-N-propyl-1,4-dioxaspiro[4.5]decan-2-methanamin,
   8-Hydroxychinolinsulfat,
   9H-Xanthen-9-carbonsäure-2-[(phenylamino)-carbonyl]-hydrazid,
   bis-(1-Methylethyl)-3-methyl-4-[(3-methylbenzoyl)-oxy]-2,5-thiophendicarboxylat,
   cis-1-(4-Chlorphenyl)-2-(1H-1,2,4-triazol-l-yl)-cycloheptanol,
   cis-4-[3-[4-(1,1-Dimethylpropyl)-phenyl-2-methylpropyl]-2,6-dimethyl-morpholin-hydrochlorid,
   Ethyl-[(4-chlorphenyl)-azo]-cyanoacetat,
   Kaliumhydrogencarbonat,
   Methantetrathiol-Natriumsalz,
   Methyl-1-(2,3-dihydro-2,2-dimethyl-1H-inden-1-yl)-1H-imidazol-5-carboxylat,
   Methyl-N-(2,6-dimethylphenyl)-N-(5-isoxazolylcarbonyl)-DL-alaninat,
   Methyl-N-(chloracetyl)-N-(2,6-dimethylphenyl)-DL-alaninat,
   N-(2,3-Dichlor-4-hydroxyphenyl)-1-methyl-cyclohexancarboxamid.
   N-(2,6-Dimethylphenyl)-2-methoxy-N-(tetrahydro-2-oxo-3-furanyl)-acetamid,
   N-(2,6-Dimethylphenyl)-2-methoxy-N-(tetrahydro-2-oxo-3-thienyl)-acetamid,
   N-(2-Chlor-4-nitrophenyl)-4-methyl-3-nitro-benzolsulfonamid,
   N-(4-Cyclohexylphenyl)-1,4,5,6-tetrahydro-2-pyrimidinamin,
   N-(4-Hexylphenyl)-1,4,5,6-tetrahydro-2-pyrimidinamin,
   N-(5-Chlor-2-methylphenyl)-2-methoxy-N-(2-oxo-3-oxazolidinyl)-acetamid,
   N-(6-Methoxy)-3-pyridinyl)-cyclopropancarboxamid,
   N-[2,2,2-Trichlor-1-[(chloracetyl)-amino]-ethyl]-benzamid,
   N-[3-Chlor-4,5-bis-(2-propinyloxy)-phenyl]-N'-methoxy-methanimidamid,
   N-Formyl-N-hydroxy-DL-alanin -Natriumsalz,
   O,O-Diethyl-[2-(dipropylamino)-2-oxoethyl]-ethylphosphoramidothioat,
   O-Methyl-S-phenyl-phenylpropylphosphoramidothioate,
   S-Methyl-1,2,3-benzothiadiazol-7-carbothioat,
   spiro[2H]-1-Benzopyran-2,1'(3'H)-isobenzofuran]-3'-on,
**Bakterizide:**
   Bronopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.
**Insektizide / Akarizide / Nematizide:**
   Abamectin, Acephat, Acrinathrin, Alanycarb, Aldicarb, Alphamethrin, Amitraz, Avermectin, AZ 60541, Azadirachtin, Azinphos A, Azinphos M, Azocyclotin,
   Bacillus thuringiensis, 4-Bromo-2-(4-chlorphenyl)-1-(ethoxymethyl)-5-(trifluoromethyl)-1H-pyrrole-3-carbonitrile, Bendiocarb, Benfuracarb, Bensultap, Betacyfluthrin, Bifenthrin, BPMC, Brofenprox, Bromophos A, Bufencarb, Buprofezin, Butocarboxim, Butylpyridaben,
   Cadusafos, Carbaryl, Carbofuran, Carbophenothion, Carbosulfan, Cartap, Chloethocarb, Chlorethoxyfos, Chlorfenapyr, Chlorfenvinphos, Chlorfluazuron, Chlormephos, N-[(6-Chloro-3-pyridinyl)-methyl]-N'-cyano-N-methyl-ethanimidamide, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cyperrnethrin, Cyromazin,
   Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat, Dimethylvinphos, Dioxathion, Disulfoton,
   Edifenphos, Emamectin, Esfenvalerat, Ethiofencarb, Ethion, Ethofenprox, Ethoprophos, Etrimphos,
   Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Fluazuron, Flucycloxuron, Flucythrinat, Flufenoxuron, Flufenprox, Fluvalinate, Fonophos, Formothion, Fosthiazat, Fubfenprox, Furathiocarb,
   HCH, Heptenophos, Hexaflumuron, Hexythiazox,
   Imidacloprid, Iprobenfos, Isazophos, Isofenphos, Isoprocarb, Isoxathion, Ivermectin,
   Lamda-cyhalothrin, Lufenuron,
   Malathion, Mecarbam, Mevinphos, Mesulfenphos, Metaldehyd, Methacrifos, Methamidophos, Methidathion, Methiocarb, Methomyl, Metolcarb, Milbemectin, Monocrotophos, Moxidectin,
   Naled, NC 184, Nitenpyram
   Omethoat, Oxamyl, Oxydemethon M, Oxydeprofos,
   Parathion A, Parathion M, Permethrin, Phenthoat, Phorat, Phosalon, Phosmet, Phosphamidon, Phoxim, Pirimicarb, Pirimiphos M, Pirimiphos A, Profenophos, Promecarb, Propaphos, Propoxur, Prothiophos, Prothoat, Pymetrozin, Pyrachlophos, Pyridaphenthion, Pyresmethrin, Pyrethrum, Pyridaben, Pyrimidifen, Pyriproxifen,
   Quinalphos,
   Salithion, Sebufos, Silafluofen, Sulfotep, Sulprofos,
   Tebufenozide, Tebufenpyrad, Tebupirimiphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
   Vamidothion, XMC, Xylylcarb, Zetamethrin.

Auch eine Mischung mit anderen bekannten Wirkstoffen, wie Herbiziden oder mit Düngemitteln und Wachstumsregulatoren ist möglich.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Wirkstoffe als Fungizide können die Aufwandmengen je nach Applikationsart innerhalb eines größeren Bereiches variiert werden. Bei der Behandlung von Pflanzenteilen liegen die Aufwandmengen an Wirkstoff im allgemeinen zwischen 0,1 und 10.000 g/ha, vorzugsweise zwischen 10 und 1.000 g/ha. Bei der Saatgutbehandlung liegen die Aufwandmengen an Wirkstoff im allgemeinen zwischen 0,001 und 50 g pro Kilogramm Saatgut, vorzugsweise zwischen 0,01 und 10 g pro Kilogramm Saatgut. Bei der Behandlung des Bodens liegen die Aufwandmengen an Wirkstoff im allgemeinen zwischen 0,1 und 10.000 g/ha, vorzugsweise zwischen 1 und 5.000 g/ha.

### Herstellungsbeispiele:

### Beispiel (1)

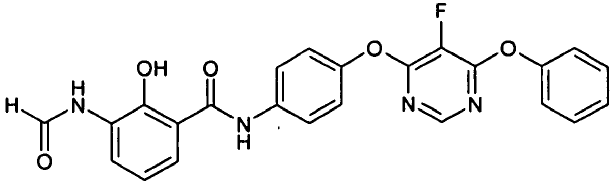

### Verfahren c)

1,67 g (3,02 mMol) 2-Benzyloxy-N-[4-(5-fluoro-6-phenoxy-pyrimidin-4-yloxy)-phenyl]-3-nitro-benzamid werden in 20 ml Ameisensäure mit 0,4 g Raney-Nickel 24 Stunden bei 40°C gerührt. Die Mischung wird auf Wasser gegeben und mehrfach mit Essigsäureethylester extrahiert. Die vereinigten organischen Phasen werden über Natriumsulfat getrocknet und bei vermindertem Druck eingeengt. Der Rückstand wird mit Cyclohexan/Essigester (1:1) an Kieselgel chromatografiert. Man erhält 0,4 g (35 % der Theorie) N-[4-(5-Fluor-6-phenoxy-pyrimidin-4-yloxy)-phenyl]-3-formylamino-2-hydroxy-benzamid als farbloses Öl.
HPLC: logP = 3,31

### Beispiel (1)

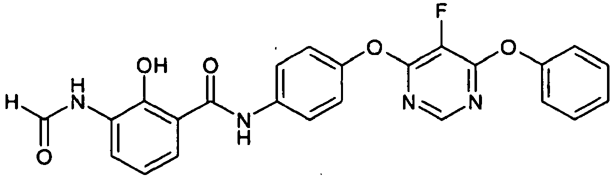

### Verfahren b)

0,5 g (1,08 mMol) N-[4-(5-Fluor-6-phenoxy-pyrimidin-4-yloxy)-phenyl]-2-hydroxy-3-nitrobenzamid werden in 10 ml Ameisensäure mit 0,5 g Raney-Nickel 24 Stunden bei 40°C gerührt. Die Mischung wird auf Wasser gegeben und mehrfach mit Essigsäureethylester extrahiert. Die vereinigten organischen Phasen werden über Natriumsulfat getrocknet und bei vermindertem Druck eingeengt. Der Rückstand wird mit Cyclohexan/Essigester (1:1) an Kieseiget chromatografiert. Man erhält 0,4 g (81 % der Theorie) N-[4-(5-Fluor-6-phenoxy-pyrimidin-4-yloxy)-phenyl]-3-formylamino-2-hydroxy-benzamid als farbloses Öl.
HPLC: logP = 3,31

### Herstellung von Verbindungen der Formel (IV)

### Beispiel (IV-1):

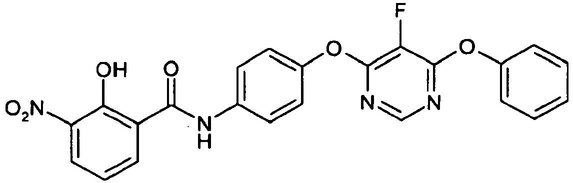

### Verfahren f)

Eine Mischung aus 1,7 g (9 mMol) 3-Nitrosalicylsäure und 2,7 g (9 mMol) 4-(5-Fluor-6-phenoxy-pyrimidin-4-yloxy)-phenylamin in 20 ml Toluol wird unter Rühren auf 50°C erhitzt. Hierzu gibt man 0,5 g Phosphortrichlorid und erhitzt weitere 4 Stunden unter Rückfluß zum Sieden. Nach dem Abkühlen wird das Gemisch eingeengt und der Rückstand mit Dichlormethan extrahiert. Die organische Phase wird mehrfach mit Wasser gewaschen und über Natriumsulfat getrocknet. Nach dem Abdestillieren des Lösungsmittels erhält man 1,7 g (41% der Theorie) N-[4-(5-Fluor-6-phenoxy-pyrimidin-4-yloxy)-phenyl]-2-hydroxy-3-nitrobenzamid.
Massenspektrum: m/e = 462 (M⁺)

### Herstellung von Verbindungen der Formel (V)

### Beispiel (V-1):

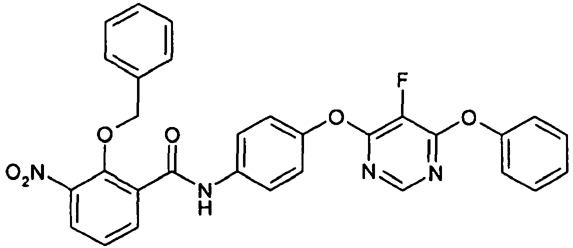

### Verfahren g)

Zu einer Lösung von 4,3 g (18 mMol) 2-Benzyloxy-3-nitro-benzoylchlorid in 20 ml Dichlormethan tropft man bei 0°C eine Lösung von 2,4 g (0,011 Mol) Triethylamin und 5,3 g (18 mMol) 4-(5-Fluor-6-phenoxy-pyrimidin-4-yloxy)-phenylamin in 30 ml Dichlormethan und rührt noch 2 Stunden ohne weitere Kühlung. Die Mischung wird filtriert, das Filtrat mit Wasser gewaschen, über Natriumsulfat getrocknet und bei vermindertem Druck eingeengt. Der Rückstand wird mit Cyclohexan/Essigester (2: 1) an Kieselgel chromatografiert. Man erhält 2,8 g (30% der Theorie) 2-Benzyloxy-N-[4-(5-fluoro-6-phenoxy-pyrimidin-4-yloxy)-phenyl]-3-nitro-benzamid als Öl.
HPLC: logP = 4,56

### Herstellung von Verbindungen der Formel (IX-a)

### Beispiel (IX-a-1):

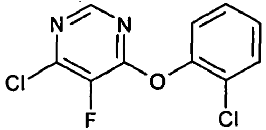

### Verfahren j)

Zu einer Mischung von 5 g (0,0295 Mol) 4,6-Dichlor-5-fluorpyrimidin und 5 g (0,036 Mol) Kaliumcarbonat in 25 ml Acetonitril wird auf 50 °C erwärmt. Bei dieser Temperatur tropft man innerhalb von 6 Stunden eine Lösung von 3,8 g (0,03 Mol) 2-Chlorphenol in 25 ml Acetonitril und rührt weitere 6 Stunden bei dieser Temperatur. Nach dem Abkühlen wird das Lösungsmittel bei vermindertem Druck abdestilliert und der Rückstand mit Wasser aufgenommen und mehrmals mit jeweils 40 ml Dichlormethan extrahiert. Die organische Phase wird mit 10%iger Natronlauge gewaschen, über Natriumsulfat getrocknet und wiederum bei vermindertem Druck eingeengt. Man erhält 6,9 g (86,7% der Theorie) 4-Chlor-6-(2-chlorphenoxy)-5-fluorpyrimidin.
¹H-NMR: δ = 7,24-7,53 (m, 4H); 8,29 (s, 1H) ppm

### Beispiel (IX-a-2):

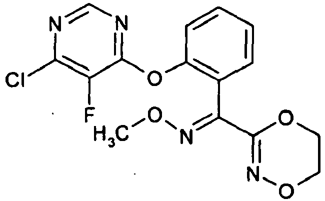

### Verfahren j)

Zu einer Mischung von 3 g (0,018 Mol) 4,6-Dichlor-5-fluorpyrimidin und 2,7 g (0,018 Mol) Kaliumcarbonat in 30 ml Acetonitril wird auf 70 °C erwärmt. Bei dieser Temperatur tropft man innerhalb von 2 Stunden eine Lösung von 3,8 g (0,03 Mol) (5,6-Dihydro-[1,4,2]dioxazin-3-yl)-(2-hydroxy-phenyl)-methanon-O-methyl-oxim in 20 ml Acetonitril und erhitzt weitere 16 Stunden unter Rückfluß. Nach dem Abkühlen wird das Lösungsmittel bei vermindertem Druck abdestilliert und der Rückstand mit Wasser aufgenommen und mehrmals mit jeweils 40 ml Dichlormethan extrahiert. Die organische Phase wird mit 10%iger Natronlauge gewaschen, über Natriumsulfat getrocknet und wiederum bei vermindertem Druck eingeengt. Man erhält 7 g (86,7% der Theorie) [2-(6-Chlor-5-fluorpyrimidin-4-yloxy)-phenyl]-(5,6-dihydro-[1,4,2]dioxazin-3-yl)-methanon-O-methyloxim.
¹H-NMR: δ = 3,83 (s, 3H); 4,14 (m, 2H); 4,45 (m, 2H); 7,39-7,54 (m, 4H); 8,32 (s, 1 H) ppm

### Herstellung von 4,6-Dichlor-5-fluorpyrimidin

### Beispiel XIII):

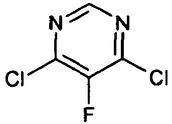

### Verfahren k)

Zu 30 ml Phosphoroxychlorid wird innerhalb von 30 Minuten 7,8 g (0,064 Mol) N,N-Dimethylanilin getropft und 10 Minuten bei 25 °C gerührt. Anschließend gibt man 8,3 g 5-Fluorpyrimidin-4,6-diol zu und erhitzt 15 Stunden unter Rückfluß. Nach dem Abkühlen wird das überschüssige Phosphoroxychlorid im Wasserstrahlvakuum abdestilliert und der Rückstand einer Vakuumdestillation unterworfen. Man erhält 11,2 g (100 % der Theorie) 4,6-Dichlor-5-fluorpyrimidin vom Siedepunkt 58 - 60 °C bei 14 mbar.

### Herstellung von Verbindungen der Formel (VII-a)

### Beispiel (VII-a-1):

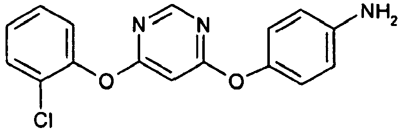

### Verfahren i)

Eine Mischung aus 2 g (9 mMol) 4-(4-Aminophenoxy)-6-chlor-pyrimidin, 1,1 g (9 mMol) 3-Chlorphenol und 2 g (18 mMol) getrocknetes Kaliumcarbonat in 20 ml Acetonitril wird 4 Stunden unter Rückfluß erhitzt. Anschließend wird die Mischung filtriert und das Filtrat bei vermindertem Druck eingeengt. Der Rückstand wird in Dichlormethan aufgenommen, mit Wasser gewaschen, die organische Phase über Natriumsulfat getrocknet und wiederum bei vermindertem Druck eingeengt. Man erhält 1,3 g (46 % der Theorie) 4-(4-Aminophenoxy)-6-(2-chlorphenoxy)-pyrimidin.
HPLC: logP = 1,96

### Herstellung von Verbindungen der Formel (XI)

### Beispiel (XI-1):

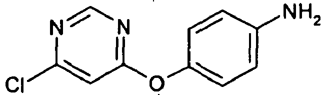

### Verfahren 1)

Eine Mischung aus 50 g (0,3 Mol) 4,6-Dichlorpyrimidin, 37 g (0,3 Mol) 4-Aminophenol, 80 g (0,6 Mol) getrocknetes Kaliumcarbonat und 50 mg Kupfer-II-bromid in 600 ml Acetonitril wird 4 Stunden unter Rückfluß erhitzt. Anschließend wird die Mischung filtriert und das Filtrat bei vermindertem Druck eingeengt. Der Rückstand wird in Dichlormethan aufgenommen, mit Wasser gewaschen, die organische Phase über Natriumsulfat getrocknet und wiederum bei vermindertem Druck eingeengt. Man erhält 60 g (58 % der Theorie) 4-(4-Aminophenoxy)-6-chlor-pyrimidin.
HPLC: logP = 0,65

### Beispiel (VII-a-2):

### Verfahren h)

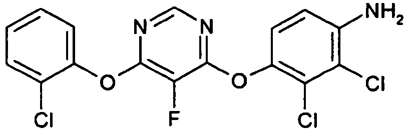

2,7 g 6-Chlor-5-fluor-4-(2-chlorphenyloxy)pyrimidin und 1,5 g Kaliumcarbonat werden in 20 ml Acetonitril vorgelegt und unter Rückfluß innerhalb von 1,5 Stunden mit 1,8 g 2,3-Dichlor-4-aminophenol versetzt. Man erhitzt weitere 12 Stunden unter Rückfluß, kühlt ab, entfernt das Lösungsmittel im Vakuum und nimmt den Rückstand in 45 ml Dichlormethan auf. Nach dem Waschen mit 0.25 N wässriger Natronlauge versetzt man die Lösung mit 5 g Aktivkohle, filtriert und destilliert das Lösungsmittel ab. Der Rückstand wird an Kieselgel mit Petrolether/Essigester (2: 1) chromatographiert.
Man erhält 2,7 g (64,3 %) an 2,3-Dichlor-4-[6-(2-chlorphenoxy)-5-fluorpyrimidin-4-yloxy]-phenylamin vom Schmelzpunkt 162-163°C.

Analog Beispiel 1, sowie entsprechend der allgemeinen Beschreibung der erfindungsgemäßen Verfahren b) und c) wurden die in der nachfolgenden Tabelle 5 genannten Verbindungen der Formel (I-f) erhalten:

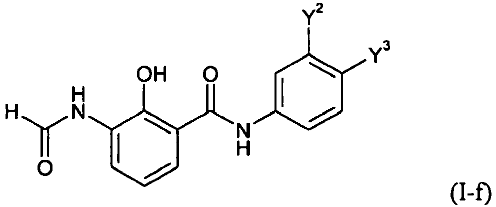

**Tabelle 5**

| Beispiel | Y² | Y³ | logP |
|---|---|---|---|
| 2 | -H | 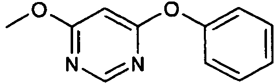 | 2,96 |
| 3 | -H | 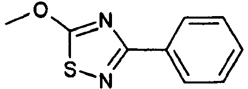 | 3,62 |
| 4 | -H | 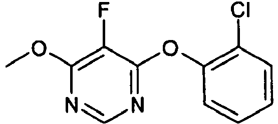 | 3,61 |
| 5 | -CH₃ | 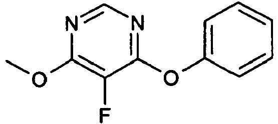 | 3,35 |
| 6 | -H | 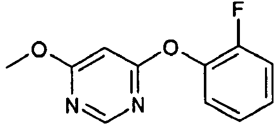 | 3,07 |
| 7 | -H | 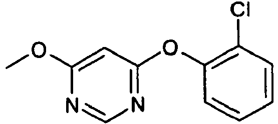 | 3,26 |
| 8 | -H | 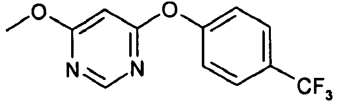 | 3,21 |
| 9 | -H | 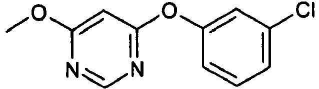 | 3,86 |
| 10 | -H | 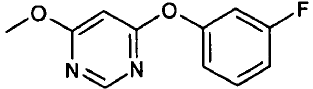 | 3,09 |
| 11 | -H | 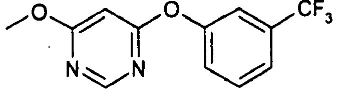 | 3,58 |
| 12 | -H | 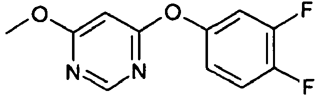 | 3,19 |
| 13 | -H | 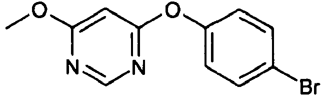 | 3,53 |
| 14 | -H | 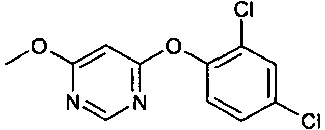 | 3,86 |
| 15 | -H | 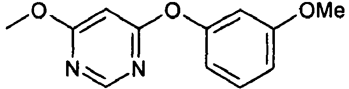 | 2,95 |
| 16 | -H | 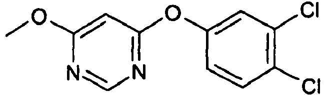 | 3,86 |
| 17 | -H | 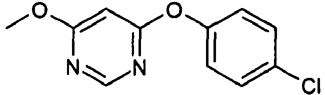 | 3,39 |
| 18 | -H | 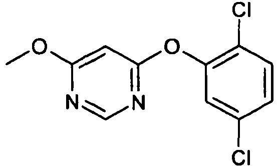 | 3,79 |
| 19 | -H | 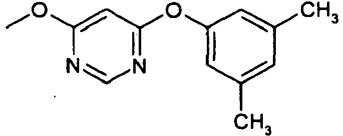 | 3,61 |
| 20 | -H | 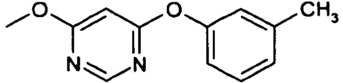 | 3,27 |
| 21 | -H | 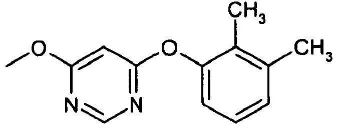 | 3,59 |
| 22 | -H | 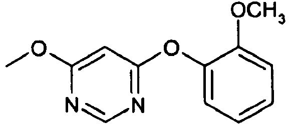 | 2,82 |
| 23 | -H | 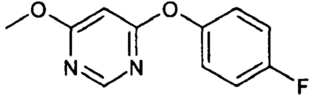 | 3,00 |
| 24 | -H | 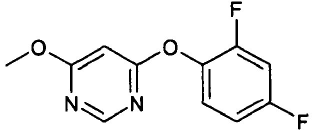 | 3,17 |
| 25 | -H | 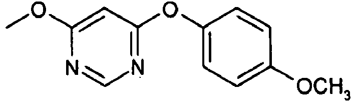 | 2,88 |
| 26 | -H | 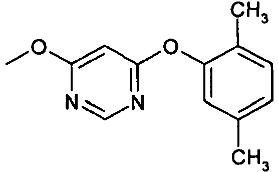 | 3,55 |
| 27 | -H | 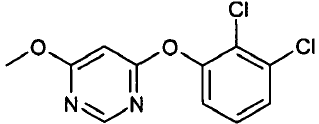 | 4,09 |
| 28 | -H | 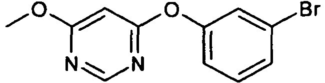 | 3,58 |
| 29 | -H | 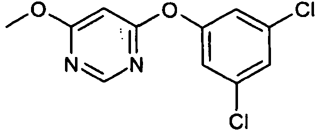 | 4,10 |
| 30 | -H | 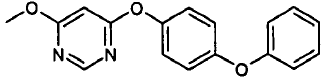 | 3,88 |
| 31 | -H | 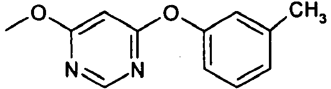 | 3,20 |
| 32 | -H | 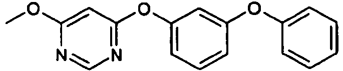 | 3,90 |

Analog Beispiel (IV-1), sowie entsprechend der allgemeinen Beschreibung des erfindungsgemäßen Verfahrens f) wurden die in der nachfolgenden Tabelle 6 genannten Verbindungen der Formel (IV-a) erhalten:

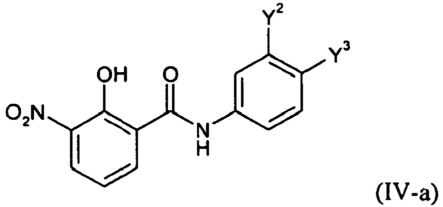

**Tabelle 6**

| Beispiel | Y² | Y³ | phys. Daten |
|---|---|---|---|
| (IV-2) | -H | 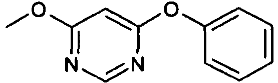 | MS*: m/e = 444 (M⁺) |

| | | | |
|---|---|---|---|
| * Massenspektrum | | | |

Analog Beispiel (V-1), sowie entsprechend der allgemeinen Beschreibung des erfindungsgemäßen Verfahrens g) wurden die in der nachfolgenden Tabelle 7 genannten Verbindungen der Formel (VI-a) erhalten:

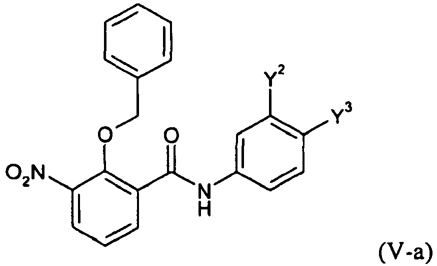

**Tabelle 7**

| Beispiel | Y² | Y³ | logP |
|---|---|---|---|
| (V-2) | -H | 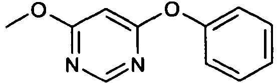 | 4,24 |
| (V-3) | -H | 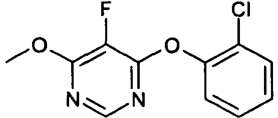 | 4,83 |
| (V-4) | -H | 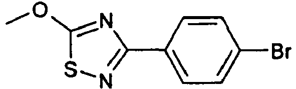 | 5,75 |
| (V-5) | -H | 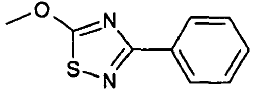 | 4,99 |
| (V-6) | -H | 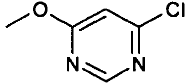 | 3,77 |
| (V-7) | -CH₃ | 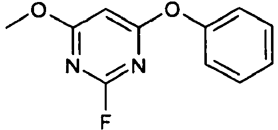 | 4,65 |
| (V-8) | -H | 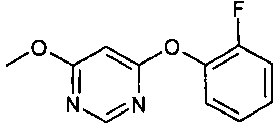 | 4,33 |
| (V-9) | -H | 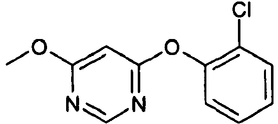 | 4,55 |
| (V-10) | -H | 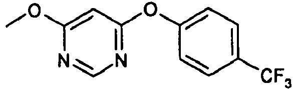 | 4,55 |
| (V-11) | -H | 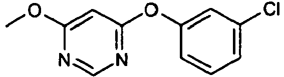 | 4,73 |
| (V-12) | -H | 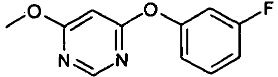 | 4,35 |
| (V-13) | -H | 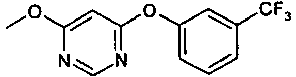 | 4,79 |
| (V-14) | -H | 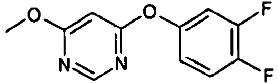 | 4,42 |
| (V-15) | -H | 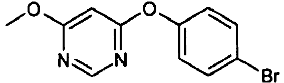 | 4,80 |
| (V-16) | -H | 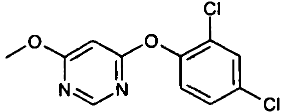 | 5,12 |
| (V-17) | -H | 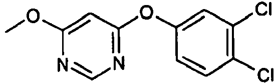 | 5,05 |
| (V-18) | -H | 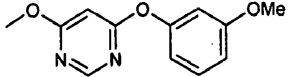 | 4,21 |
| (V-19) | -H | 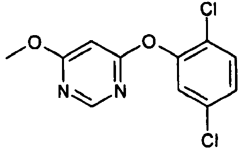 | 5,05 |
| (V-20) | -H | 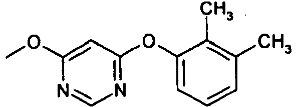 | 4,77 |
| (V-21) | -H | 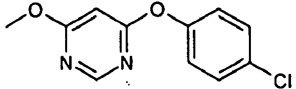 | 4,69 |
| (V-22) | -H | 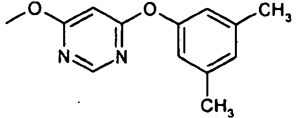 | 4,90 |
| (V-23) | -H | 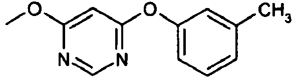 | 4,56 |
| (V-24) | -H | 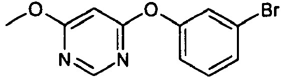 | 4,84 |
| (V-25) | -H | 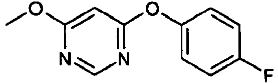 | 4,28 |
| (V-26) | -H | 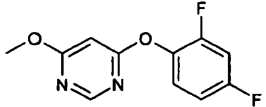 | 4,42 |
| (V-27) | -H | 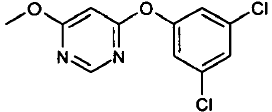 | 5,39 |
| (V-28) | -H | 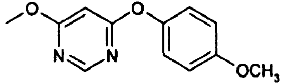 | 4,15 |
| (V-29) | -H | 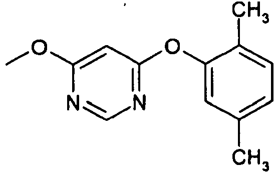 | 4,85 |
| (V-30) | -H | 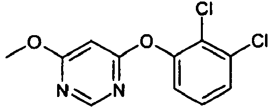 | 4,96 |
| (V-31) | -H | 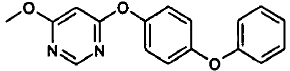 | 5,06 |
| (V-32) | -H | 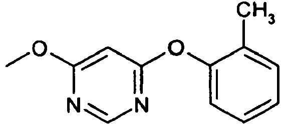 | 4,52 |
| (V-33) | -H | 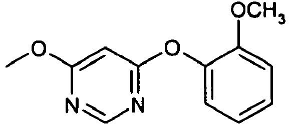 | 4,09 |
| (V-34) | -H | 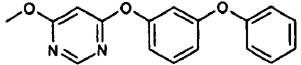 | 5,12 |

Analog Beispiel (VII-a-1) und (VII-a-2), sowie entsprechend der allgemeinen Beschreibung der erfindungsgemäßen Verfahren h) und i) wurden die in der nachfolgenden Tabelle 8 genannten Verbindungen der Formel (VII-b) erhalten:

**Tabelle 8**

| | | | | | | | |
|---|---|---|---|---|---|---|---|
| 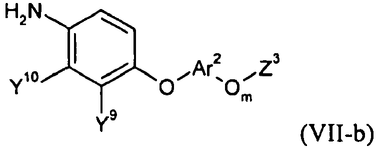 | | | | | | | |

| Bsp-Nr | Y¹⁰ | Y⁹ | Ar² | m | Z³ | Fp.: | LogP |
|---|---|---|---|---|---|---|---|
| VII-a-3 | -H | -H | 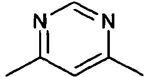 | 1 | Phenyl | 130 | 1,53 |
| VII-a-4 | -H | -H | 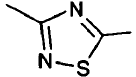 | 0 | Phenyl | | 2,57 |
| VII-a-5 | -H | -H | 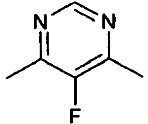 | 1 | Phenyl | | 1,88 |
| VII-a-6 | -H | -CH3 | 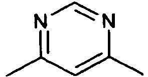 | 1 | Phenyl | | 2,32 |
| VII-a-7 | -H | -H | 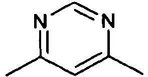 | 1 | 4-Chlorphenyl | | 2,12 |
| VII-a-8 | -H | -H | 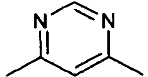 | 1 | 3,4-Difluorphenyl | | 4,55 |
| VII-a-9 | -H | -H | 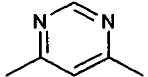 | 1 | 4-Bromphenyl | | 2,02 |
| VII-a-10 | -H | -H | 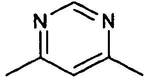 | 1 | 2,4-Dichlorphenyl | | 2,33 |
| VII-a-11 | -H | -H | 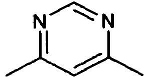 | 1 | 3,4-Dichlorphenyl | | 2,48 |
| VII-a-12 | -H | -H | 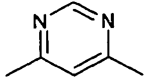 | 1 | 3-Methoxyphenyl | | 1,61 |
| VII-a-13 | -H | -H | 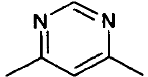 | 1 | 4-Trifluormethylphenyl | | 2,21 |
| VII-a-14 | -H | -H | 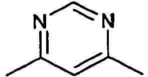 | 1 | 3-Trifluormethylphenyl | | 2,18 |
| VII-a-15 | -H | -H | 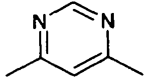 | 1 | 2-Fluorphenyl | | 1,61 |
| VII-a-16 | -H | -H | 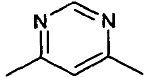 | 1 | 3-Fluorphenyl | | 1,46 |
| VII-a-17 | -H | -H | 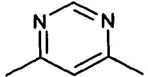 | 1 | 3-Tolyl | | 2,12 |
| VII-a-18 | -H | -H | 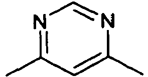 | 1 | 2,5-Dichlorphenyl | | 2,26 |
| VII-a-19 | -H | -H | 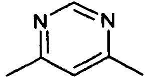 | 1 | 2,3-Dimethylphenyl | | 1,98 |
| VII-a-20 | -H | -H | 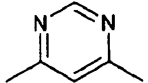 | 1 | 3,5-Dimethylphenyl | | 2,12 |
| VII-a-21 | -H | -H | 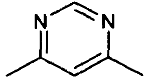 | 1 | 2,4-Difluorphenyl | | 1,76 |
| VII-a-22 | -H | -H | 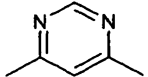 | 1 | 3,5-Dichlorphenyl | | 2,6 |
| VII-a-23 | -H | -H | 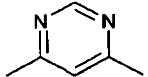 | 1 | 4-Methoxyphenyl | | 1,52 |
| VII-a-24 | -H | -H | 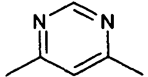 | 1 | 4-Fluorphenyl | | 1,59 |
| VII-a-25 | -H | -H | 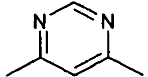 | 1 | 3-Bromphenyl | | 2,05 |
| VII-a-26 | -H | -H | 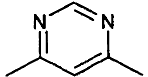 | 1 | 2,3-Dichlorphenyl | | 2,26 |
| VII-a-27 | -H | -H | 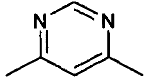 | 1 | 2,5-Dimethylphenyl | | 2,02 |
| VII-a-28 | -H | -H | 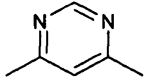 | 1 | 4-Phenoxyphenyl | | 2,55 |
| VII-a-29 | -H | -H | 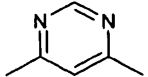 | 1 | 2-Methoxyphenyl | | 1,46 |
| VII-a-30 | -H | -H | 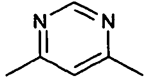 | 1 | 2-Tolyl | | 1,71 |
| VII-a-31 | -H | -H | 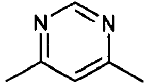 | 1 | 3-Phenoxyphenyl | | 2,54 |
| VII-a-32 | -H | -H | 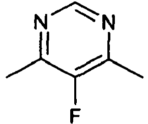 | 1 | 2-Chlorphenyl | | 3,61 |
| VII-a-33 | -H | -H | 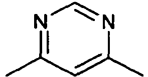 | 1 | 2,6-Dimethylphenyl | | |
| VII-a-34 | -H | -H | 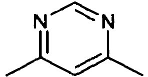 | 1 | 2,6-Dichlorphenyl | | |

### Anwendungsbeispiele:

### Beispiel A

### Phytophthora-Test (Tomate) / protektiv

| | |
|---|---|
| Lösungsmittel: | 47 Gewichtsteile Aceton |
| Emulgator: | 3 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension von Phytophthora infestans inokuliert. Die Pflanzen werden dann in einer Inkubationskabine bei ca. 20°C und 100 % relativer Luftfeuchtigkeit aufgestellt.

3 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Bei diesem Test zeigen z.B. die folgenden Verbindungen der Herstellungsbeispiele 1, 2 und 4 bei einer beispielhaften Wirkstoffaufwandmenge von 100 g/ha einen Wirkungsgrad von über 80% im Vergleich zur unbehandelten Kontrolle.

### Beispiel B

### Venturia-Test (Apfel) / protektiv

| | |
|---|---|
| Lösungsmittel: | 47 Gewichtsteile Aceton |
| Emulgator: | 3 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Konidiensuspension des Apfelschorferregers ***Venturia inaequalis*** inokuliert und verbleiben dann 1 Tag bei ca. 20°C und 100 % relativer Luftfeuchtigkeit in einer Inkubationskabine.

Die Pflanzen werden dann im Gewächshaus bei ca. 21°C und einer relativen Luftfeuchtigkeit von ca. 90 % aufgestellt.

12 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Bei diesem Test zeigen z.B. die folgenden Verbindungen der Herstellungsbeispiele 1 und 2 bei einer beispielhaften Wirkstoffaufwandmenge von 100 g/ha einen Wirkungsgrad von über 80% im Vergleich zur unbehandelten Kontrolle.

## Patentansprüche

1. 4,6-Dichlor-5-fluorpyrimidin der Formel (XIII),

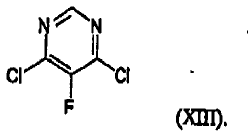

2. Verfahren zur Herstellung von 4,6-Dichlor-5-fluorpyrimidin, **dadurch gekennzeichnet, daß** man (Verfahren k) 5-Fluor-6-hydroxy-4(1H)-pyrimidinon (XIV) mit einem Chlorierungsmittel, gegebenenfalls in Gegenwart eines Verdünnugsmittels und gegebenenfalls in Gegenwart eines Katalysators, umsetzt.

3. Verbindungen der Formel 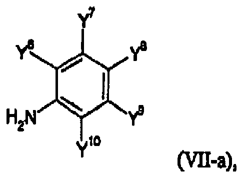 in welcher
Y⁶, Y⁹ und Y¹⁰ gleich oder verschieden sind und für Wasserstoff, Halogen, Cyano, Alkyl, Halogenalkyl, Alkoxy oder Halogenalkoxy mit jeweils 1-4 Kohlenstoffatomen stehen,
Y⁷ und Y⁸ verschieden sind und und für Wasserstoff, Halogen, Cyano, Alkyl, Halogenalkyl, Alkoxy oder Halogenalkoxy mit jeweils 1-4 Kohlenstoffatomen stehen, und einer der Reste
Y⁷ oder Y⁸ für eine Gruppierung 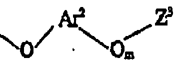 steht,
worin
m für 0 oder 1 steht,
Ar² für 1,2,4-Oxadiazol-3,5-diyl, 1,2,4-Thiadiazol-3,5-diyl oder für gegebenenfalls in 5-Stellung durch Halogen substituiertes Pyrimidin-4,6-diyl steht und
Z³ für gegebenenfalls substituiertes Aryl steht.

4. Verbindungen der Formel (VII-a) gemäß Anspruch 3,
in welcher
Y⁶, Y⁹ und Y¹⁰ gleich oder verschieden sind und für Wasserstoff, Halogen, Cyano, Alkyl, Halogenalkyl, Alkoxy oder Halogenalkoxy mit jeweils 1-4 Kohlenstoffatomen stehen,
Y⁷ und Y⁸ verschieden sind und und für Wasserstoff, Halogen, Cyano, Alkyl, Halogenalkyl, Alkoxy oder Halogenalkoxy mit jeweils 1-4 Kohlenstoffatomen stehen, und einer der Reste
Y⁷ oder Y⁸ für eine Gruppierung 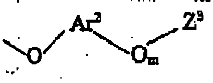 steht,
worin
m für 0 oder 1 steht,
Ar² für 1,2,4-Oxadiazol-3,5-diyl, 1,2,4-Thadiazol-3,5-diyl oder für gegebenenfalls in 5-Stellung durch Fluor oder Chlor substituiertes Pyrimidin-4,6-diyl steht und
Z³ für jeweils gegebenenfalls einfach oder mehrfach, gleich oder verschieden substituiertes Phenyl oder Naphthyl steht, wobei die möglichen Substituenten vorzugsweise aus der nachstehenden Aufzählung ausgewählt sind:
Halogen, Cyano, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 11 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl oder Alkylsulfonyloxy, mit jeweils 1 bis 6 Kohlensffatomen in den einzelnen Alkylteilen;
jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen;
Cycloalkyl mit 3 bis 6 Kohlenstoffatomen;
jeweils gegebenenfalls einfach oder mehrfach, gleich oder verschieden durch Halogen und/oder geradkettiges oder verzweigtes Alkyl mit 1 bis 4 Kohlenstoffatomen substituiertes Phenyl oder Phenoxy;
Heterocyclyl oder Heterocyclyl-methyl mit jeweils 3 bis 7 Ringgliedern, von denen jeweils 1 bis 3 gleiche oder verschiedene Heteroatome sind - insbesondere Stickstoff, Sauerstoff und/oder Schwefel - , oder
eine Gruppierung 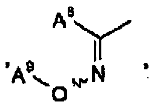 worin
A⁸ für Alkyl mit 1 bis 4 Kohlehstoffatomen oder Cycloalkyl mit 1 bis 6 Kohlenstoffatomen steht und
A⁹ für gegebenenfalls durch Cyano, Alkoxy, Alkylthio, Alkylamino, Dialkylamino oder Phenyl substituiertes Alkyl mit 1 bis 4 Kohlenstoffatomen, Alkenyl oder Alkinyl mit jeweils 2 bis 4 Kohlenstoffatomen steht, oder
eine Gruppierung, 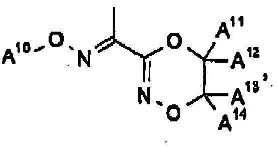 in welcher
A¹⁰ für Alkyl mit 1 bis 4 Kohlenstoffatomen steht und
A¹¹, A¹², A¹³ und A¹⁴ gleich oder verschieden sind und unabhängig voneinander für Wasserstoff, Alkyl oder Hydroxyalkyl mit jeweils 1 bis 4 Kohlenstoffatomen oder Halogenalkyl mit 1 bis 4 Kohlenstoffatomen und 1 bis fünf gleichen oder verschiedenen Halogenatomen stehen, oder
A¹¹ und A¹² oder A¹¹ und A¹³ oder A¹³ und A¹⁴ gemeinsam mit den jeweiligen Kohlenstoffatomen, an die sie gebunden sind, einen cycloaliphatischen Ring mit fünf, sechs oder sieben Kohlenstoffatomen bilden.

5. Verbindungen der Formel (VII-a) gemäß Anspruch 3,
in welcher
Y⁶, Y⁹ und Y¹⁰ gleich oder verschieden sind und für Wasserstoff. Fluor, Chlor, Brom, Cyano, Methyl, Ethyl, Methoxy, Trifluormethyl oder Difluormethoxy stehen, und
Y⁷ und Y⁸ verschieden sind und für Wasserstoff, Fluor, Chlor, Brom, Cyano, Methyl, Ethyl, Methoxy, Trifluormethyl oder Difluormethoxy stehen, und einer der Reste
Y⁷ oder Y⁸ für eine Gruppierung 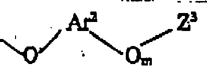 steht,
worin
m für 0 oder 1 steht,
Ar² für 1,2,4-Oxadiazol-3,5-diyl, 1,2,4-Thiadiazol-3,5-diyl oder für gegebenenfalls in 5-Stellung durch Fluor oder Chlor substituiertes Pyrimidin-4,6-diyl steht und
Z³ für jeweils gegebenenfalls einfach bis dreifach, gleich oder verschieden substituiertes Phenyl steht, wobei die möglichen Substituenten vorzugsweise aus der nachstehenden Aufzählung ausgewählt sind:
Fluor, Chlor, Brom, Cyano, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Trifluormethylthio, Difluorchlormethylthio, Trifluormethylsulfinyl oder Trifluormethylsulfonyl, Methoxycarbonyl, Ethoxycarbonyl,
jeweils gegebenenfalls einfach bis vierfach, gleich oder verschieden durch Fluor, Chlor, Methyl, Trifluormethyl oder Ethyl substituiertes, jeweils zweifach verknüpftes Methylendioxy, Ethylendioxy
Phenyl, 4-Chlorphenyl, 4-Methylphenyl, Phenol, 4-Chlorphenoxy, 4-Methylphenoxy, Cyclopropyl. Cyclobutyl, Cyclopentyl oder Cyclohexyl, oder
eine Gruppierung, 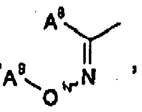 worin
A⁸ für Methyl, Ethyl, n- oder propyl, n-, i-, s- oder t-Butyl, Cyclopropyl oder Cyclobutyl steht,
A⁹ für Methyl, Ethyl, n- oder i-Propyl, n-, i-, s- oder t-Butyl, Alkyl, Propargyl, But-2-en-1-yl, 2-Methyl-prop-1-en-3-yl, Cyanmethyl, Methoxymethyl, Ethoxymethyl, Methoxyethyl, Ethoxyethyl, Methylthiomethyl, Ethylthiomethyl, Methylthioethyl, Ethylthioethyl, Dimethylaminomethyl, Dimethylaminoethyl, Methylaminomethyl, Methylaminoethyl oder Benzyl steht, oder
eine Gruppierung, 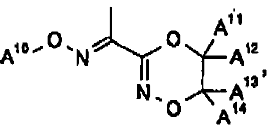 in welcher
A¹⁰ für Methyl oder Ethyl steht und
A¹¹, A¹², A¹³ und A¹⁴ gleich oder verschieden sind und unabhängig voneinander für Wasserstoff, Methyl, Ethyl, n- oder i-Propyl, n-, i-, s- oder t-Butyl, Hydroxyethyl, Trifluormethyl oder Trifluorethyl stehen, oder
A¹¹ und A¹² oder A¹¹ und A¹³ oder A¹³ und A¹⁴ gemeinsam mit den jeweiligen Kohlenstoffatomen, an die sie gebunden sind, einen cycloaliphatischen Ring mit fünf, sechs oder sieben Kohlenstoffatomen bilden.

6. Verfahren zur Herstellung von Verbindungen der Formel (VII-a) wie in Anspruch 3 definiert, **dadurch gekennzeichnet, daß** man Diazole der allgemeinen Formel (VIII) oder Halogenpyrimidine der allgemeinen Formel (IX), 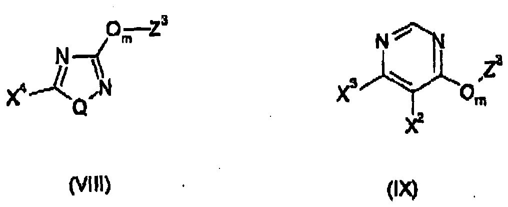 worin
m für 0 oder 1 steht,
X² für Wasserstoff, Fluor oder Chlor steht,
X³ für Chlor oder Fluor steht,
X⁴ für Methylsulfonyl, Chlor oder Brom steht und
Q für Sauerstoff oder Schwefel steht, und
Z³ die in Anspruch 3 angegebene Bedeutung hat,
mit einem Aminophenol der Formel, 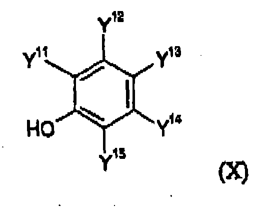 in welcher
Y¹¹, Y¹⁴ und Y¹⁵gleich oder verschieden sind und für Wasserstoff, Halogen, Cyano, Alkyl, Halogenalkyl, Alkoxy oder Halogenalkoxy mit jeweils 1-4 Kohlenstoffatomen stehen,
Y¹² and Y¹³ verschieden sind und für Wasserstoft, Halogen, Cyano, Alkyl, Halogen alkyl, Alkoxy, oder Halogenalkoxy mit jeweils 1-4 Kohlenstoffatomen stehen und einer der Reste
Y¹² oder Y¹³ für Amino steht,
gegebenenfalls in Gegenwart eines Verdünnungsmittels, gegebenenfalls in Gegenwart eines Säureakzeptors und gegebenenfalls in Gegenwart eines Katalysators, untsetzt, oder
(Verfahren i) Aminophenoxypyrimidine der allgemeinen Formel (XI), 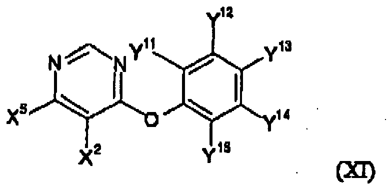 in welcher
X², Y¹¹, Y¹², Y¹³, Y¹⁴ und Y¹⁵ die oben angegebenen Bedeutungen haben und
X⁵ für Chlor oder Fluor steht,
mit einem Phenol der allgemeinen Formel (XII),
Z³-OH (XII)
in welcher
Z³ die oben angegebene Bedeutung hat,
gegebenenfalls in Gegenwart eines Verdünnungsmittels, gegebenenfalls in Gegenwart eines Säureakzeptors und gegebenenfalls in Gegenwart eines Katalysators, umsetzt,

7. Verbindungen der Formel (IX-a) 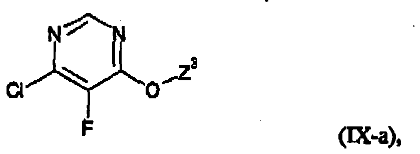 in welcher
Z³ die inden Ansprüchen 3 bis 5 angegebenen Bedeutungen hat, wobei die Verbindung 2-[(6-Chlor-5-fluor-4-pyrimidinyl)-oxy]-a-(methoxy-methylen)-benzoesäuremethylester, ausgenommen ist.

8. Verfahren zur Herstellung von Verbindungen der Formel (IX-a) wie in Anspruch 7, definiert, **dadurch gekennzeichnet, daß** man (Verfahren j) Phenole der allgemeinen Formel (XII),
Z³-OH (XII)
in welcher
Z³ die in den Ansprüchen 3 bis 5 angegebenen Bedeutungen hat,
mit 4,6-Dichlor-5-fluorpyrimidin (XIII), gegebenenfalls in Gegenwart eines Verdünnungsmittels, gegebenenfalls in Gegenwart eines Säureakzeptors und gegebenenfalls in Gegenwart eines Katalysators, umsetzt.

9. Verbindungen der Formel (XI), 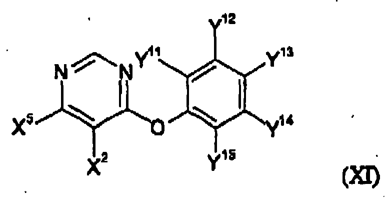 In welcher
X², X⁵, Y¹², Y¹³_{,} Y¹⁴ und Y¹⁵ die in Anspruch 6 angegebenen Bedeutungen haben.

10. Verfahren zur Herstellung von Verbindungen der Formel (XI) wie in Anspruch 9 definiert, **dadurch gekennzeichnet, daß** man (Verfahren I) Trihalogenpyrimidine der Formel (XV), 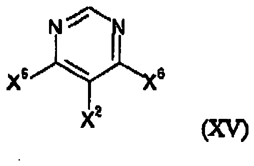 in welcher
X² und X⁵ die in Anspruch 6 angegebenen Bedeutungen haben und
X⁶ für Fluor oder Chlor steht,
mit einem Aminophenol der Formel (X),
gegebenenfalls in Gegenwart eines Verdünnungsmittels, gegebenenfalls in Gegenwart eines Säureakzeptors und gegebenenfalls in Gegenwart eines Katalysators, umsetzt.

## Claims

1. 4,6-Dichloro-5-fluoropyrimidine of the formula (XIII).

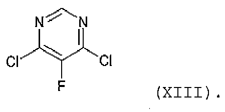

2. Process for preparing 4,6-dichloro-5-fluoropyrimidine, **characterized in that** (process k) 5-fluoro-6-hydroxy-4(1H)-pyrimidinone (XIV) is reacted with a chlorinating agent, if appropriate in the presence of a diluent and if appropriate in the presence of a catalyst.

3. Compounds of the formula 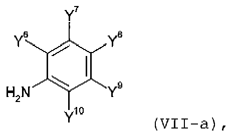 in which
Y⁶, Y⁹ and Y¹⁰ are identical or different and each represents hydrogen, halogen, cyano, alkyl, halogenoalkyl, alkoxy or halogenoalkoxy having in each case 1-4 carbon atoms,
Y⁷ and Y⁸ are different and each represents hydrogen, halogen, cyano, alkyl, halogenoalkyl, alkoxy or halogenoalkoxy having in each case 1-4 carbon atoms, and one of the radicals
Y⁷ or Y⁸ represents a grouping 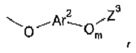 in which
m represents 0 or 1,
Ar² represents 1,2,4-oxadiazole-3,5-diyl, 1,2,4-thiadiazole-3,5-diyl or represents pyrimidine-4,6-diyl which is optionally substituted in the 5 position by halogen and
Z³ represents optionally substituted aryl.

4. Compounds of the formula (VII-a) according to Claim 3,
in which
Y⁶, Y⁹ and Y¹⁰ are identical or different and each represents hydrogen, halogen, cyano, alkyl, halogenoalkyl, alkoxy or halogenoalkoxy having in each case 1-4 carbon atoms,
Y⁷ and Y⁸ are different and each represents hydrogen, halogen, cyano, alkyl, halogenoalkyl, alkoxy or halogenoalkoxy having in each case 1-4 carbon atoms, and one of the radicals
Y⁷ or Y⁸ represents a grouping 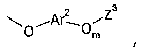 in which
m represents 0 or 1,
Ar² represents 1,2,4-oxadiazole-3,5-diyl, 1,2,9-thiadiazole-3,5-diyl or represents pyrimidine-4,6-diyl which is optionally substituted in the 5 position by fluorine and chlorine and
Z³ represents phenyl or naphthyl, each of which is optionally mono- or polysubstituted by identical or different substituents, where the possible substituents are preferably selected from the list below:
halogen, cyano, amino, hydroxyl, formyl, carboxyl, carbamoyl, thiocarbamoyl;
in each case straight-chain or branched alkyl, alkoxy, alkylthio, alkylsulphinyl or alkylsulphonyl having in each case 1 to 6 carbon atoms;
in each case straight-chain or branched alkenyl or alkenyloxy having in each case 2 to 6 carbon atoms;
in each case straight-chain or branched halogenoalkyl, halogenoalkoxy, halogenoalkylthio, halogenoalkylsulphinyl or halogenoalkylsulphonyl having in each case 1 to 6 carbon atoms and 1 to 13 identical or different halogen atoms;
in each case straight-chain or branched halogenoalkenyl or halogenoalkenyloxy having in each case 2 to 6 carbon atoms and 1 to 11 identical or different halogen atoms;
in each case straight-chain or branched alkylamino, dialkylamino, alkylcarbonyl, alkylcarbonyloxy, alkoxycarbonyl or alkylsulphonyloxy, having in each case 1 to 6 carbon atoms in the individual alkyl moieties;
in each case doubly attached alkylene or dioxyalkylene having in each case 1 to 6 carbon atoms and being in each case optionally mono- or polysubstituted by identical or different substituents selected from the group consisting of halogen, straight-chain or branched alkyl having 1 to 4 carbon atoms and straight-chain or branched halogenoalkyl having 1 to 4 carbon atoms and 1 to 9 identical or different halogen atoms;
cycloalkyl having 3 to 6 carbon atoms;
phenyl or phenoxy, each of which is optionally mono- or polysubstituted by identical or different substituents selected from the group consisting of halogen and straight-chain or branched alkyl having 1 to 4 carbon atoms;
heterocyclyl or heterocyclyl-methyl having in each case 3 to 7 ring members, 1 to 3 of which are in each case identical or different heteroatoms - in particular nitrogen, oxygen and/or sulphur-, or
a grouping 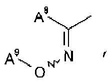 in which
A⁸ represents alkyl having 1 to 4 carbon atoms or cycloalkyl having 1 to 6 carbon atoms and
A⁹ represents optionally cyano-, alkoxy-, alkylthio-, alkylamino-, dialkylamino- or phenyl-substituted alkyl having 1 to 4 carbon atoms, alkenyl or alkinyl having in each case 2 to 4 carbon atoms, or
a grouping 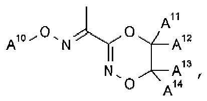 in which
A¹⁰ represents alkyl having 1 to 4 carbon atoms and
A¹¹, A¹², A¹³ and A¹⁴ are identical or different and, independently of one another, each represents hydrogen, alkyl or hydroxyalkyl having in each case 1 to 4 carbon atoms or halogenoalkyl having 1 to 4 carbon atoms and 1 to five identical or different halogen atoms, or
A¹¹ and A¹² or A¹¹ and A¹³ or A¹³ andA¹⁴ together with the respective carbon atoms to which they are attached form a cycloaliphatic ring having five, six or seven carbon atoms.

5. Compounds of the formula (VII-a) according to Claim 3,
in which
Y⁶, Y⁹ and Y¹⁰ are identical or different and each represents hydrogen, fluorine, chlorine, bromine, cyano, methyl, ethyl, methoxy, trifluoromethyl or difluoromethoxy, and
Y⁷ and Y⁸ are different and each represents hydrogen, fluorine, chlorine, bromine, cyano, methyl, ethyl, methoxy, trifluoromethyl or difluoromethoxy, and one of the radicals
Y⁷ or Y⁸ represents a grouping 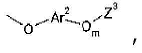 in which
m represents 0 or 1,
Ar² represents 1,2,4-oxadiazole-3,5-diyl, 1,2,4-thiadiazole-3,5-diyl or represents pyrimidine-4,6-diyl which is optionally substituted in the 5 position by fluorine or chlorine and
Z³ represents phenyl which is in each case optionally mono- to trisubstituted by identical or different substituents, where the possible substituents are preferably selected from the list below: fluorine, chlorine, bromine, cyano, methyl, ethyl, n- or i-propyl, n-, i-, s- or t-butyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, trifluoromethylthio, difluorochloromethylthio, trifluoromethylsulphinyl or trifluoromethylsulphonyl, methoxycarbonyl, ethoxycarbonyl,
in each case doubly attached methylenedioxy, ethylenedioxy, each of which is optionally mono- to tetrasubstituted by identical or different substituents selected from the group consisting of fluorine, chlorine, methyl, trifluoromethyl or ethyl
phenyl, 4-chlorophenyl, 4-methylphenyl, phenoxy, 4-chlorophenoxy, 4-methylphenoxy, cyalopropyl, cyclobutyl, cyclopentyl or cyclohexyl, or
a grouping 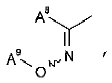 in which
A⁸ represents methyl, ethyl, n- or i-propyl, n-, i-, s- or t-butyl, cyclopropyl or cyclobutyl,
A⁹ represents methyl, ethyl, n- or i-propyl, n-, i-, s- or t-butyl, allyl, propargyl, but-2-en-1-yl, 2-methyl-prop-1-en-3-yl, cyanomethyl, methoxymethyl, ethoxymethyl, methoxyethyl, ethoxyethyl, methylthiomethyl, ethylthiomethyl, methylthioethyl, ethylthioethyl, dimethylaminomethyl, dimethylaminoethyl, methylaminomethyl, methylaminoethyl or benzyl, or
a grouping 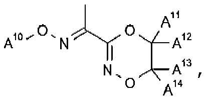 in which
A¹⁰ represents methyl or ethyl and
A¹¹, A¹², A¹³ and A¹⁴ are identical or different and, independently of one another, each represents hydrogen, methyl, ethyl, n- or i-propyl, n-, i-, s- or t-butyl, hydroxymethyl, trifluoromethyl or trifluoroethyl, or
A¹¹ and A¹² or A¹¹ and A¹³ or A¹³ and A¹⁴ together with the respective carbon atoms to which they are attached form a cycloaliphatic ring having five, six or seven carbon atoms.

6. Process for preparing compounds of the formula (VII-a) as defined in Claim 3, **characterized in that** diazoles of the general formula (VIII) or halogenopyrimidines of the general formula (IX) 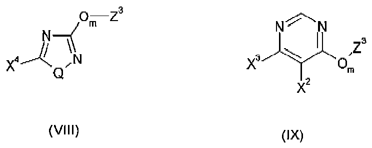 in which
m represents 0 or 1,
X² represents hydrogen, fluorine or chlorine,
X³ represents chlorine or fluorine,
X⁴ represents methylsulphonyl, chlorine or bromine and
Q represents oxygen or sulphur, and
Z³ is as defined in Claim 3,
are reacted with an aminophenol of the formula 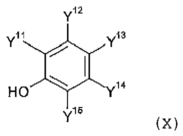 in which
Y¹¹, Y¹⁴ and Y¹⁵ are identical or different and each represents hydrogen, halogen, cyano, alkyl, halogenoalkyl, alkoxy or halogenoalkoxy having in each case 1-4 carbon atoms,
Y¹² and Y¹³ are different and each represents hydrogen, halogen, cyano, alkyl, halogenoalkyl, alkoxy or halogenoalkoxy having in each case 1-4 carbon atoms, and one of the radicals
Y¹² or Y¹³ represents amino,
if appropriate in the presence of a diluent, if appropriate in the presence of an acid acceptor and if appropriate in the presence of a catalyst, or
(process i) aminophenoxypyrimidines of the general formula (XI) 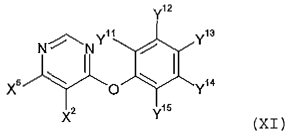 in which
X², Y¹¹, Y¹², Y¹³, Y¹⁴ and Y¹⁵ are each as defined above and
X⁵ represents chlorine or fluorine,
are reacted with a phenol of the general formula (XII),
Z³-OH (XII)
in which
Z³ is as defined above,
if appropriate in the presence of a diluent, if appropriate in the presence of an acid acceptor and if appropriate in the presence of a catalyst.

7. Compounds of the formula (IX-a) 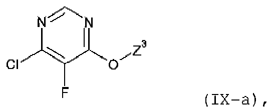 in which
Z³ is as defined in Claims 3 to 5, except for the compound methyl 2-[(6-chloro-5-fluoro-4-pyrimidinyl)-oxy]-a-(methoxymethylene)-benzoate.

8. Process for preparing compounds of the formula (IX-a) as defined in Claim 7, **characterized in**
**that** (process j) phenols of the general formula (XII)
Z³-OH (XII)
in which
Z³ is as defined in Claims 3 to 5,
are reacted with 4,6-dichloro-5-fluoropyrimidine (XIII), if appropriate in the presence of a diluent, if appropriate in the presence of an acid acceptor and if appropriate in the presence of a catalyst.

9. Compounds of the formula (XI), 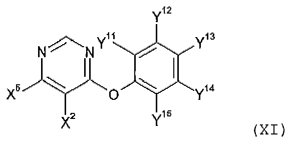 in which
X², X⁵, Y¹¹, Y¹², Y¹³, Y¹⁴ and Y¹⁵ are each as defined in Claim 6.

10. Process for preparing compounds of the formula (XI) as defined in Claim 9, **characterized in that** (process 1) trihalogenopyrimidines of the formula (XV), 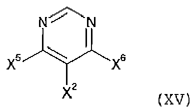 in which
X² and X⁵ are each as defined in Claim 6 and
X⁶ represents fluorine or chlorine,
are reacted with an aminophenol of the formula (X),
if appropriate in the presence of a diluent, if appropriate in the presence of an acid acceptor and if appropriate in the presence of a catalyst.

## Revendications

1. 4,6-dichloro-5-fluoropyrimidine de formule (XIII),

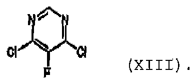

2. Procédé pour la préparation de 4,6-dichloro-5-fluoropyrimidine, **caractérisé en ce qu'**on fait réagir (procédé k) de la 5-fluoro-6-hydroxy-4(1H)-pyrimidinone (XIV) avec un agent de chloration, éventuellement en présence d'un diluant et éventuellement en présence d'un catalyseur.

3. Composés de formule 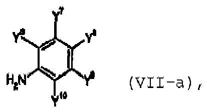 dans laquelle
Y⁶, Y⁹ et Y¹⁰ sont identiques ou différents et représentent un atome d'hydrogène ou d'halogène, un groupe cyano, alkyle, halogénoalkyle, alcoxy ou halogénoalcoxy ayant chacun de 1 à 4 atomes de carbone,
Y⁷ et Y⁸ sont différents et représentent un atome d'hydrogène ou d'halogène, un groupe cyano, alkyle, halogénoalkyle, alcoxy ou halogénoalcoxy ayant chacun de 1 à 4 atomes de carbone, et l'un des radicaux
Y⁷ ou Y⁸ représente un groupement 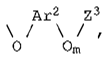 dans lequel
m représente 0 ou 1,
Ar² représente le groupe 1,2,4-oxadiazole-3,5-diyle, 1,2,4-thiadiazole-3,5-diyle ou un groupe pyrimidine-4,6-diyle éventuellement substitué en position 5 par un halogène et
Z³ représente un groupe aryle éventuellement substitué.

4. Composés de formule (VII-a) selon la revendication 3,
dans lesquels
Y⁶, Y⁹ et Y¹⁰ sont identiques ou différents et représentent un atome d'hydrogène ou d'halogène, un groupe cyano, alkyle, halogénoalkyle, alcoxy ou halogénoalcoxy ayant chacun de 1 à 4 atomes de carbone,
Y⁷ et Y⁸ sont différents et représentent un atome d'hydrogène ou d'halogène, un groupe cyano, alkyle, halogénoalkyle, alcoxy ou halogénoalcoxy ayant chacun de 1 à 4 atomes de carbone, et l'un des radicaux
Y⁷ ou Y⁸ représente un groupement 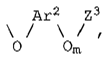 dans lequel
m représente 0 ou 1,
Ar² représente le groupe 1,2,4-oxadiazole-3,5-diyle, 1,2,4-thiadiazole-3,5-diyle ou un groupe pyrimidine-4,6-diyle éventuellement substitué en position 5 par le fluor ou le chlore et
Z³ représente un groupe phényle ou naphtyle éventuellement substitués chacun une ou plusieurs fois par des substituants identiques ou différents, les substituants possibles étant choisis de préférence dans l'énumération suivante :
halogène, cyano, amino, hydroxy, formyle, carboxy, carbamoyle, thiocarbamoyle ;
alkyle, alcoxy, alkylthio, alkylsulfinyle ou alkylsulfonyle chacun à chaîne droite ou ramifiée, ayant chacun de 1 à 6 atomes de carbone ;
alcényle ou alcényloxy chacun à chaîne droite ou ramifiée, ayant chacun de 2 à 6 atomes de carbone :
halogénoalkyle, halogénoalcoxy, halogénoalkylthio, halogénoakylsulfinyle ou halogénoalkylsulfonyle, chacun à chaîne droite ou ramifiée, ayant chacun de 1 à 6 atomes de carbone et de 1 à 13 atomes d'halogène identiques ou différents ;
halogénoalcényle ou halogénoalcényloxy chacun à chaîne droite ou ramifiée, ayant chacun de 2 à 6 atomes de carbone et de 1 à 11 atomes d'halogène identiques ou différents ;
alkylamino, dialkylamino, alkylcarbonyle, alkylcarbonyloxy, alcoxycarbonyle ou alkylsulfonyloxy chacun à chaîne droite ou ramifiée, ayant chacun de 1 à 6 atomes de carbone dans les fragments alkyle individuels ;
alkylène ou dioxyalkylène ayant chacun de 1 à 6 atomes de carbone, chacun doublement lié, chacun portant éventuellement un ou plusieurs substituants, identiques ou différents, halogène et/ou alkyle à chaîne droite ou ramifiée ayant de 1 à 4 atomes de carbone et/ou halogénoalkyle à chaîne droite ou ramifiée ayant de 1 à 4 atomes de carbone et de 1 à 9 atomes d'halogène identiques ou différents ;
cycloalkyle ayant de 3 à 6 atomes de carbone ;
phényle ou phénoxy chacun portant éventuellement un ou plusieurs substituants, identiques ou différents, halogène et/ou alkyle à chaîne droite ou ramifiée ayant de 1 à 4 atomes de carbone ;
hétérocyclyle ou hétérocyclyl-méthyle ayant chacun de 3 à 7 chaînons formant le cycle, dont dans chaque cas 1 à 3 sont des hétéroatomes identiques ou différents - en particulier d'azote, d'oxygène et/ou de soufre, ou
un groupement, 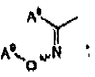 dans lequel
A⁸ représente un groupe alkyle ayant de 1 à 4 atomes de carbone ou cycloalkyle ayant de 1 à 6 atomes de carbone et
A⁹ représente un groupe alcényle ou alcynyle ayant chacun de 2 à 4 atomes de carbone, alkyle ayant de 1 à 4 atomes de carbone, éventuellement substitué par cyano, alcoxy, alkylthio, alkylamino, dialkylamino ou phényle, ou
un groupement, 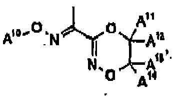 dans lequel
A¹⁰ représente un groupe alkyle ayant de 1 à 4 atomes de carbone et
A¹¹, A¹², A¹³ et A¹⁴ sont identiques ou différents et représentent indépendamment les uns des autres un atome d'hydrogène, un groupe alkyle ou hydroxyalkyle ayant chacun de 1 à 4 atomes de carbone ou halogénoalkyle ayant de 1 à 4 atomes de carbone et de 1 à cinq atomes d'halogène identiques ou différents, ou
A¹¹ et A¹² ou A¹¹ et A¹³ ou A¹³ et A¹⁴ forment ensemble avec les atomes de carbone respectifs, auxquels ils sont liés, un cycle cycloaliphatique ayant cinq, six ou sept atomes de carbone.

5. Composés de formule (VII-a) selon la revendication 3,
dans lesquels
Y⁶, Y⁹ et Y¹⁰ sont identiques ou différents et représentent un atome d'hydrogène, de fluor, chlore, brome, un groupe cyano, méthyle, éthyle, méthoxy, trifluorométhyle ou difluorométhoxy, et
Y⁷ et Y⁸ sont différents et représentent un atome d'hydrogène, de fluor, chlore, brome, un groupe cyano, méthyle, éthyle, méthoxy, trifluorométhyle ou difluorométhoxy, et l'un des radicaux
Y⁷ ou Y⁸ représente un groupement 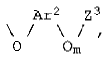 dans lequel
m représente 0 ou 1,
Ar² représente le groupe 1,2,4-oxadiazole-3,5-diyle, 1,2,4-thiadiazole-3,5-diyle ou un groupe pyrimidine-4,6-diyle éventuellement substitué en position 5 par le fluor ou le chlore et
Z³ représente un groupe phényle portant éventuellement un à trois substituants identiques ou différents, les substituants possibles étant choisis dans l'énumération suivante :
fluoro, chloro, bromo, cyano, méthyle, éthyle, n- ou isopropyle, n-, iso-, sec- ou tert-butyle, méthoxy, éthoxy, n- ou isopropoxy, méthylthio, éthylthio, n- ou isopropylthio, méthylsulfinyle, éthylsulfinyle, méthylsulfonyle ou éthylsulfonyle, trifluorométhyle, trifluoroéthyle, difluorométhoxy, trifluorométhoxy, difluorochlorométhoxy, trifluoroéthoxy, difluorométhylthio, trifluorométhylthio, difluorochlorométhylthio, trifluorométhylsulfinyle ou trifluorométhylsulfonyle, méthoxycarbonyle, éthoxycarbonyle,
méthylènedioxy, éthylènedioxy chacun doublement lié, chacun portant éventuellement un à quatre substituants, identiques ou différents, fluoro, chloro, méthyle, trifluorométhyle ou éthyle
phényle, 4-chlorophényle, 4-méthylphényle, phénoxy, 4-chlorophénoxy, 4-méthylphénoxy, cyclopropyle, cyclobutyle, cyclopentyle ou cyclohexyle, ou
un groupement, 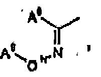 dans lequel
A⁸ représente le groupe méthyle, butyle, n-ou isopropyle, n-, iso-, sec- ou tert-butyle, cyclopropyle ou cyclobutyle,
A⁹ représente le groupe méthyle, éthyle, n-ou isopropyle, n-, iso-, sec- ou tert-butyle, allyle, propargyle, but-2-én-1-yle, 2-méthyl-prop-1-én-3-yle, cyanométhyle, méthoxyméthyle, éthoxyméthyle, méthoxyéthyle, éthoxyéthyle, méthylthiométhyle, éthylthiométhyle, méthylthioéthyle, éthylthioéthyle, diméthylaminométhyle, diméthylaminoéthyle, méthylaminométhyle, méthylaminoéthyle ou benzyle, ou
un groupement, 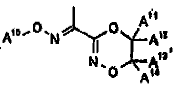 dans lequel
A¹⁰ représente le groupe méthyle ou éthyle et
A¹¹, A¹², A¹³ et A¹⁴ sont identiques ou différents et représentent indépendamment les uns des autres un atome d'hydrogène, le groupe méthyle, éthyle, n- ou isopropyle, n-, iso-, sec-ou tert-butyle, hydroxyméthyle, trifluorométhyle ou trifluoroéthyle, ou
A¹¹ et A¹² ou A¹¹ et A¹³ ou A¹³ et A¹⁴ forment ensemble avec les atomes de carbone respectifs, auxquels ils sont liés, un cycle cycloaliphatique ayant cinq, six ou sept atomes de carbone.

6. Procédé pour la préparation de composés de formule (VII-a) telle que définie dans la revendication 3, **caractérisé en ce qu'**on fait réagir des diazoles de formule générale (VIII) ou des halogénopyrimidines de formule générale (IX), 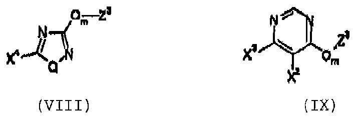 où
m représente 0 ou 1,
x² représente un atome d'hydrogène, de fluor ou de chlore,
X³ représente un atome de chlore ou de fluor,
X⁴ représente le groupe méthylsulfonyle, un atome de chlore ou de brome et
Q représente un atome d'oxygène ou de soufre, et
Z³ a la signification indiquée dans la revendication 3,
avec un aminophénol de formule, 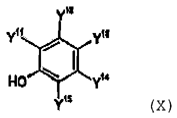 dans laquelle
Y¹¹, Y¹⁴ et Y¹⁵ sont identiques ou différents et représentent un atome d'hydrogène ou d'halogène, un groupe cyano, alkyle, halogénoalkyle, alcoxy ou halogénoalcoxy ayant chacun de 1 à 4 atomes de carbone,
Y¹² et Y¹³ sont différents et représentent un atome d'hydrogène ou d'halogène, un groupe cyano, alkyle, halogénoalkyle, alcoxy ou halogénoalcoxy ayant chacun de 1 à 4 atomes de carbone, et l'un des radicaux
Y¹² ou Y¹³ représente un groupe amino,
éventuellement en présence d'un diluant, éventuellement en présence d'un capteur d'acide et éventuellement en présence d'un catalyseur, ou
(procédé i) on fait réagir des aminophénoxypyrimidines de formule générale (XI), 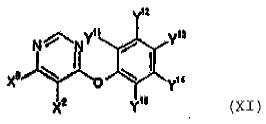 dans laquelle
X², Y¹¹ Y¹², Y¹³, Y¹⁴ et Y¹⁵ ont les significations indiquées plus haut et
X⁵ représente un atome de chlore ou de fluor,
avec un phénol de formule générale (XII),
Z³-OH (XII)
dans laquelle
Z³ a la signification indiquée plus haut,
éventuellement en présence d'un diluant, éventuellement en présence d'un capteur d'acide et éventuellement en présence d'un catalyseur.

7. Composés de formule (IX-a) 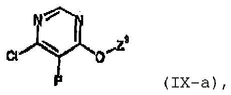 dans laquelle
Z³ a les significations indiquées dans les revendications 3 à 5, à l'exclusion du composé 2-[(6-chloro-5-fluoro-4-pyrimidinyl)-oxy]-a-(méthoxyméthylène)-benzoate de méthyle.

8. Procédé pour la préparation de composés de formule (IX-a) telle que définie dans la revendication 7, **caractérisé en ce qu'**on fait réagir (procédé j) des phénols de formule générale (XII),
Z³-OH (XII)
dans laquelle
Z³ a les significations indiquées dans les revendications 3 à 5,
avec de la 9,6-dichloro-5-fluoropyrimidine (XIII), éventuellement en présence d'un diluant, éventuellement en présence d'un capteur d'acide et éventuellement en présence d'un catalyseur.

9. Composés de formule (XI), 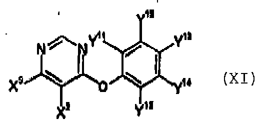 dans laquelle
X², X⁵, Y¹¹, Y¹², _{Y}¹³, Y¹⁴ et Y¹⁵ ont les significations indiquées dans la revendication 6.

10. Procédé pour la préparation de composés de formule (XI) telle que définie dans la revendication 9, **caractérisé en ce qu'**on fait réagir (procédé 1) des trihalogénopyrimidines de formule (XV), 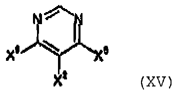 dans laquelle
X² et X⁵ ont les significations indiquées dans la revendication 6 et
X⁶ représente un atome de fluor ou de chlore,
avec un aminophénol de formule (X),
éventuellement en présence d'un diluant, éventuellement en présence d'un capteur d'acide et éventuellement en présence d'un catalyseur.
